(19) Europäisches Patentamt
European Patent Office
Office européen des brevets

(11) Veröffentlichungsnummer: **0 349 481 B1**

(12) **EUROPÄISCHE PATENTSCHRIFT**

(45) Veröffentlichungstag der Patentschrift: **02.11.95**

(21) Anmeldenummer: **89730155.2**

(22) Anmeldetag: **03.07.89**

(51) Int. Cl.$^6$: **C07J 1/00**, A61K 31/565, C07J 15/00, C07J 17/00, C07J 21/00, C07J 33/00, C07J 41/00, C07J 43/00, A61K 31/58, A61K 31/585

(54) **13-Alkyl-11Beta-phenylgonane.**

(30) Priorität: **01.07.88 DE 3822770**

(43) Veröffentlichungstag der Anmeldung:
**03.01.90 Patentblatt 90/01**

(45) Bekanntmachung des Hinweises auf die Patenterteilung:
**02.11.95 Patentblatt 95/44**

(84) Benannte Vertragsstaaten:
**AT BE CH DE ES FR GB GR IT LI LU NL SE**

(56) Entgegenhaltungen:
**EP-A- 0 057 115**
**EP-A- 0 129 499**
**EP-A- 0 190 759**
**EP-A- 0 254 670**
**WO-A-83/03099**

(73) Patentinhaber: **SCHERING AKTIENGESELL-SCHAFT**

**D-13342 Berlin (DE)**

(72) Erfinder: **Scholz, Stefan, Dr.**
**Lutzen Strasse 9**
**D-1000 Berlin 31 (DE)**
Erfinder: **Ottow, Eckhard, Dr.**
**Sonnenallee 124**
**D-1000 Berlin 44 (DE)**
Erfinder: **Neef, Günter, Dr.**
**Markgraf-Albrecht-Strasse 4**
**D-1000 Berlin 15 (DE)**
Erfinder: **Elger, Walter, Dr.**
**Schorlemer Allee 12B**
**D-1000 Berlin 33 (DE)**
Erfinder: **Beier, Sybille, Dr.**
**Uhlandstrasse 121**
**D-1000 Berlin 31 (DE)**
Erfinder: **Chwalisz, Krzysztof, Dr.**
**Luzerner Strasse 1d**
**D-1000 Berlin 45 (DE)**

**EP 0 349 481 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung bezieht sich auf 13-Alkyl-11β-phenyl-gonane der allgemeinen Formel I

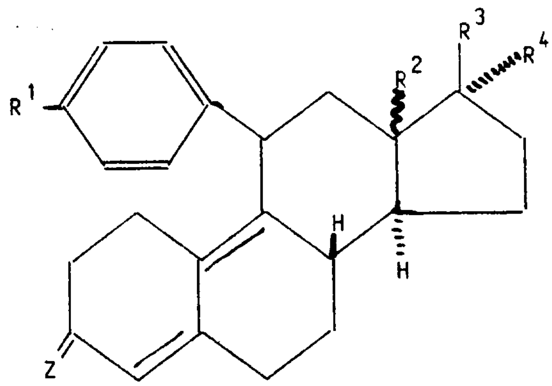

(I) ,

worin
Z für ein Sauerstoffatom oder die Hydroxyiminogruppierung N~OH,
$R^1$ entweder für
a) einen Heteroarylrest der Formel I a

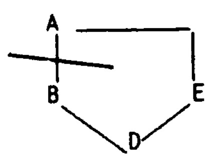

(Ia),

wobei A = N, O oder S und
B-D-E die Elementenfolge
C-C-C, N-C-C oder C-N-C, ausgenommen den 1-Pyrazolrest, bebedeuten oder
b) für einen Heteroarylrest der Formel I b

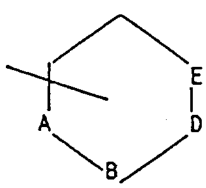

(I b) ,

wobei A = N und
B-D-E die Elementenfolge
C-C-C, N-C-C, C-N-C oder C-C-N bedeuten oder
c) für einen Cycloalkyl-, Cycloalkenyl- oder Arylrest I c oder
d) für einen geradkettigen oder verzweigten, eine oder mehrere Doppelbindungen aufweisenden Alkenylrest Id mit 2 bis 10 Kohlenstoffatomen stehen
und wobei gegebenenfalls
der Heteroarylrest der Formel I a durch einen oder mehrere Halogenreste und/oder einen oder mehrere Alkylreste mit 1 bis 3 Kohlenstoffatomen
und der Cycloalkyl-, Cycloalkenyl- oder Arylrest Ic durch eine oder mehrere Halogen-, gegebenenfalls geschützte Hydroxy- , Alkoxy-, gegebenenfalls in Form des Sulfoxids oder Sulfons und/oder des N-Oxids oxidierte Alkylthio- und/oder Dialkylaminoreste

substituiert ist/sind, und
$R^2$ für einen $\alpha$- oder $\beta$-ständigen Methyl- oder Ethylrest steht,
wobei wenn $R^2$ $\alpha$-ständig ist
$R^1$ zusätzlich für einen geradkettigen oder verzweigten Alkylrest mit 1 bis 10 Kohlenstoffatomen steht und
$R^3/R^4$

$-OR_5/-C \equiv CY$

$-C \equiv CY/-OR_5$

$-OR_5/-\underset{\underset{O}{\|}}{C}-CH_2R_6$

$-\underset{\underset{O}{\|}}{C}-CH_2R_6/-OR_5$

$-CH_3/-\underset{\underset{O}{\|}}{C}-CH_2R_6$

$-\underset{\underset{O}{\|}}{C}-CH_2R_6/-CH_3$

$-OR_5/-H$

$-H/-OR_5$

$-H/-\underset{\underset{O}{\|}}{C}-CH_2R_6$

$-\underset{\underset{O}{\|}}{C}-CH_2R_6/H$

$-OR_5/(CH_2)_o-CH_2R_7$

$-(CH_2)_o-CH_2R_7/-OR_5$

$-OR_5/-CH{=}CH-(CH_2)_kCH_2R_7$

$-CH{=}CH-(CH_2)_kCH_2R_7/-OR_5$

und

17 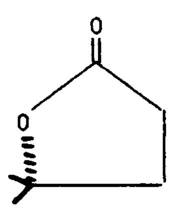 oder 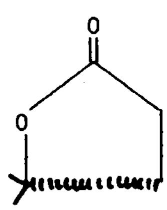

wenn $R^2$ $\beta$-ständig ist
$R^1$ zusätzlich für einen Ethylrest steht, $R^1$ aber nicht der Alkenylrest Isopropenyl sein kann und
$R^3/R^4$

$-OR_5/-C\equiv CY$

$C-CH_2R_6/-H$
||
O

$-OR_5/-C-CH_2R_6$
||
O

$-OR_5/-(CH_2)_o-CH_2R_7$

$-C-CH_2R_6/-OR_5$
||
O

$-OR_5/-CH=CH-(CH_2)_kCH_2R_7$

$-OR_5/-H$

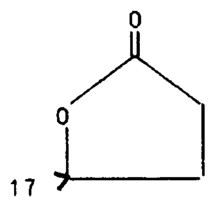

$-C-CH_2R^6/-CH_3$
||
O

mit $R_5$ in der Bedeutung eines Wasserstoffatoms oder Acylrestes mit 1 bis 4 Kohlenstoffatomen,
Y in der Bedeutung eines Wasserstoff-, Chlor-, Fluor-, Iod- oder Bromatoms, einer Alkyl-, Hydroxyalkyl-, Alkoxyalkyl- oder Acyloxyalkylgruppe mit jeweils 1 bis 4 Kohlenstoffatomen im Alkyl- bzw. Acylrest,
$R_6$ in der Bedeutung eines Wasserstoffatoms, einer Hydroxygruppe, einer Alkyl-, O-Alkyl- oder O-Acylgruppe mit jeweils 1 bis 4 Kohlenstoffatomen,
o in der Bedeutung 0, 1, 2 oder 3,
$R_7$ in der Bedeutung eines Hydroxy- oder Cyanidrestes, einer O-Alkyl- oder O-Acylgruppe mit jeweils 1 bis 4 Kohlenstoffatomen und
k in der Bedeutung 0, 1 oder 2, bedeuten,
Verfahren zu deren Herstellung, diese Verbindungen enthaltende pharmazeutische Präparate sowie die neuen Ausgangs- und Zwischenprodukte und Verfahren zu deren Herstellung.

11β-Organyl-4-estrene, die in 9, 10-Position eine zusätzliche Bindung oder eine Epoxygruppe aufweisen und im 11β-Organylrest mindestens ein N-, P- oder Si-Atom tragen, gehen aus der EP-A-0 057 115. Die beschriebenen Verbindungen besitzen antiglucocorticoide sowie antigestagene Wirkung. Änliche Verbindungen sind weiterhin von EPA 254670 und WO.A. 83/03099 bekannt.

Von den gemäß Formel I a möglichen Heteroarylresten sind der 3-Thienyl-, 3-Furyl-, und 3-Pyrrolrest bevorzugt.
Als Heteroarylreste der Formel I b kommen erfindungsgemäß insbesondere der 3- oder 4-Pyridyl-, der 5-Pyrimidin-, 4-Pyridazin- oder Pyrazinrest infrage.

Als Cycloalkyl-, Cycloalkenyl- oder Arylrest I c sind der Cyclohexyl-, der Cyclohex-1-enyl-, Cyclohex-2-enyl-, Cyclohex-3-enyl- sowie der Phenylrest besonders hervorzuheben.
Der Alkenylrest I d soll vorzugsweise bis zu 3 Doppelbindungen aufweisen.

Als Halogensubstituenten, die am Heteroarylrest der Formel I a möglich sind, sind insbesondere ein Chlor- oder Bromatom zu nennen.
Ist der Heteroarylrest der Formel I a alkylsubstituiert, so ist die Monosubstitution bevorzugt.

Der Cycloalkyl-, Cycloalkenyl- oder Arylrest I c kann durch ein oder zwei Chlor- und/oder Bromatom(e) substituiert sein. Die genannten Reste können auch durch ein oder zwei, gegebenenfalls geschützte Hydroxy- und/oder Alkoxyreste mit 1 bis 8 Kohlenstoffatomen substituiert sein.

Die Verbindungen der allgemeinen Formel I besitzen eine starke Affinität zum Gestagenrezeptor, ohne selbst gestagene Aktivität zu entfalten. Sie sind kompetitive Antagonisten des Progestgerons (Antigestagene); da sie das zur Aufrechterhaltung der Schwangerschaft erforderliche Progesteron vom Rezeptor verdrängen, sind sie zur Auslösung von Aborten und zur Einleitung der Geburt geeignet.

Neben den genannten Indikationen können die erfindungsgemäßen Verbindungen auch zur Behandlung der Endometriose, Dysmenorrhoe und hormonabhängiger Tumore, wie z.B. Mamma-Carcinom und Meningeom verwendet werden.

Zur Kennzeichnung der antigestagenen Wirkung der erfindungsgemäßen Verbindungen wurde die abortive Wirksamkeit bestimmt. Die Versuche wurden an weiblichen Ratten mit einem Gewicht von ca. 200 g durchgeführt. Nach erfolgter Anpaarung wurde der Schwangerschaftsbeginn durch Nachweis von Spermien in Vaginalabstrichen gesichert. Der Tag des Spermiennachweises gilt als Tag 1 der Gravidität ( = d 1 p.c.).
Die Behandlung der Tiere mit der jeweils zu testenden Substanz bzw. dem Lösungsmittel erfolgte an d.5 bis d.7 p.c.

An d.9 p.c. wurden die Tiere getötet und die Uteri auf Implantate und Resorptionsstellen hin untersucht. Das Fehlen von Implantaten wurde als Abort gewertet.

Als Antigestagene wurden untersucht:

A: 17α-(3-Hydroxyprop-1-(Z)-enyl)-17β-hydroxy-11β-(4-vinylphenyl)-4,9-estradien-3-on
B: 17α-(Prop-1-inyl)-17β-hydroxy-11β-(4-vinylphenyl)-4,9-estradien-3-on
C: 17β-(3-Hydroxypropyl)-17α-hydroxy-13α-methyl-11β-(4-isopropenylphenyl)-4,9-gonadien-3-on
D: 11β-(4-Ethylphenyl)-17-hydroxy-17β-(3-hydroxypropyl)-13α-methyl-4,9-gonadien-3-on
E: 17-Hydroxy-17β-(3-hydroxypropyl)-13α-methyl-11β-(4-vinylphenyl)-4,9-gonadien-3-on
F: 17-Hydroxy-17α-(3-hydroxyprop-(Z)-1-enyl)-11β-[4-(3-pyridyl)-phenyl]-4,9-estradien-3-on
G: 17-Hydroxy-17β-(3-hydroxypropyl)-13α-methyl-11β-[4-(3-pyridyl)-phenyl]-4,9-gonadien-3-on
H: 17-Hydroxy-17β-(3-hydroxypropyl)-13α-methyl-11β-[4-(2-thiazolyl)-phenyl]-4,9-gonadien-3-on
I: 17-Hydroxy-17α-(3-hydroxy-(Z)-1-enyl)-11β-[4-(3-thienyl)-phenyl]-4,9-estradien-3-on
K: 17-Hydroxy-17β-(3-hydroxypropyl)-13α-methyl-11β-[4-(3-furyl)-phenyl]-4,9-gonadien-3-on
L: 17-Hydroxy-17β-(3-hydroxypropyl)-13α-methyl-11β-[4-(3-thienyl)-phenyl]-4,9-gonadien-3-on
M: 17-Hydroxy-17α-(3-hydroxyprop-(Z)-1-enyl)-11β-[4-(3-furyl)-phenyl]-4,9-estradien-3-on
N: 17-Hydroxy-17β-(3-hydroxypropyl)-13α-methyl-11β-[4-(4-cyanophenyl)-phenyl]-4,9-gonadien-3-on
O: 17-Hydroxy-17β-(3-hydroxypropyl)-13α-methyl-11β-[4-(5-pyrimidyl)-phenyl]-4,9-gonadien-3-on
P: 17-Hydroxy-17α-(3-hydroxyprop-(Z)-1-enyl)-11β-[4-(2-thiazolyl)-phenyl]-4,9-estradien-3-on
Q: 11β-[4-(Dimethylaminophenyl]-17β-hydroxy-17α-(propin-1-yl)-4,9(10)-estradien-3-on, RU 486 (EP-A 0 057 115)

Die Testsubstanzen wurden in einem Benzylbenzoat-Rizinusöl-Gemisch (Verhältnis 1:4) gelöst. Die Behandlung erfolgte subcutan (s.c.).

Die Ergebnisse sind in Tabelle 1 zusammengestellt.

**TABELLE 1**

Abortive Wirkung der erfindungsgemäßen Verbindungen A bis P sowie der Vergleichsverbindung Q in einer frühen Phase der Gravidität von Ratten. Behandlung von d.5 bis d.7 p.c. Autopsie an d.9 p.c.

| Verbindung | Dosis mg/Tier/Tag s.c. | Abortrate n. Abort / n gesamt |
|---|---|---|
| A | 3.0 | 4/4 |
| | 1,0 | 4/4 |
| | 0,3 | 2/4 |
| B | 3.0 | 4/4 |
| | 1,0 | 3/4 |
| | 0,3 | 0/4 |
| C | 3.0 | 4/4 |
| | 1,0 | 0/4 |

Fortsetzung Tabelle 1

| Verbindung | Dosis mg/Tier/Tag s.c. | Abortrate n Abort / n gesamt |
|---|---|---|
| D | 3,0 | 3/4 |
| | 1,0 | 0/4 |
| E | 3,0 | 3/4 |
| | 1,0 | 0/4 |
| F | 3,0 | 4/4 |
| | 1,0 | 4/4 |
| | 0,3 | 0/4 |
| G | 3,0 | 4/4 |
| | 1,0 | 4/4 |
| | 0,3 | 4/4 |
| | 0,1 | 0/4 |
| H | 3,0 | 4/4 |
| | 1,0 | 3/4 |
| | 0,3 | 0/4 |
| I | 3,0 | 4/4 |
| | 1,0 | 4/4 |
| | 0,3 | 0/4 |
| K | 3,0 | 4/4 |
| | 1,0 | 4/4 |
| | 0,3 | 4/4 |
| | 0,1 | 0/4 |
| L | 3,0 | 4/4 |
| | 1,0 | 4/4 |
| | 0,3 | 0/4 |

Fortsetzung Tabelle 1

| Verbindung | Dosis mg/Tier/Tag s.c. | Abortrate n Abort / n gesamt |
|---|---|---|
| M | 3,0 | 4/4 |
| | 1,0 | 4/4 |
| | 0,3 | 4/4 |
| | 0,1 | 4/4 |
| N | 3,0 | 4/4 |
| | 1,0 | 4/4 |
| | 0,3 | 0/4 |
| O | 3,0 | 4/4 |
| | 1,0 | 4/4 |
| | 0,3 | 0/4 |
| P | 3,0 | 4/4 |
| Lösungsmittel als Kontrolle: 0,2 ml Benzylbenzoat + Rizinusöl (1:4) | - | 0/4 |

Wenn für $R^1$ in der 13α-Methylreihe ein 3-Pyridylrest (G) oder 3-Furylrest (K) oder in der 13β-Methylreihe ein Furylrest (M) steht, zeigen die erfindungsgemäßen Verbindungen sehr starke Wirksamkeit; insbesondere ist die Substitution mit einem 3-Furylrest in der 13β-Methylreihe hervorzuheben: die getestete Verbindung M ist selbst bei einer Dosis von 0,1 mg/Tier/Tag noch voll wirksam, während die als Standardsubstanz geltende Verbindung Q bereits bei einer Dosis von 0, 3 mg/Tier/Tag total unwirksam ist.

Zur Kennzeichnung der antiglucocorticoiden Wirkung wurde der Einfluß der erfindungsgemäßen Substanzen auf die Tyrosin-Aminotransferase bestimmt. Das Test-System basiert auf einer Messung der Aktivität des Leberenzyms Tyrosin Aminotransferase (TAT) in Kulturen von RHC (Rat Hepatoma Cells) Zellen. Das Enzym katalysiert den ersten Schritt in der Verstoffwechselung von Tyrosin und ist sowohl in der Leber als auch in Hepatomzellen durch Glucocorticoide induzierbar. Die Aktivität ist in Rohextrakten leicht meßbar (Granner und Tomkins. (1970) Meth. Enzymol 15. 633). Das Enzym überführt die Aminogruppe von Tyrosin auf 2-Oxoglutarsäure. Dabei entstehen Glutaminsäure und p-Hydroxyphenylpyruvat.In alkalischer Lösung wird aus p-Hydroxyphenylpyruvat der stabilere p-Hydroxybenzaldehyd gebildet, dessen Absorption bei 331 nm gemessen wird. Die TAT-Aktivität in RHC-Zellen zeigt eine dosisabhängige Induktion mit Cortisol (max. Akt. bei $10^{-6}$ M) oder Dexamethason (max. Akt. bei $10^{-7}$ M). Die Aktivität läßt sich um den Faktor 4 - 6 über den Basalwert stimulieren. Gleichzeitige Behandlung mit Corticoid und Antiglucocorticoid führt zu einer Abnahme der TAT-Aktivität.

Die erfindungsgemäßen Verbindungen A und E zeigen in diesem Test ungefähr 1%, die Verbindung F und G ungefähr 2%, die Verbindung D ungefähr 5% und die Verbindung B ungefähr 100 % der Aktivität von 11β-(4-Dimethylaminophenyl)-17β-hydroxy17α-(prop-1-inyl)-4,9-estradien-3-onRU 486 (Q), einer Substanz, die als Standard anzusehen ist (7th Int. Congress of Endocrinology July 1-7. 1984, Quebec City,

Canada; Excerpta Medica, Amsterdam-Oxford-Princeton).

Die erfindungsgemäßen Verbindungen der allgemeinen Formel I können in Form pharmazeutischer Präparate Verwendung finden. Die Herstellung der Präparate erfolgt nach an sich bekannten Methoden der Galenik durch Mischen mit organischem oder anorganischem inertem Trägermaterial, welches für die enterale, perkutane oder parenterale Applikation geeignet ist.

Die Dosierung der erfindungsgemäßen Verbindungen für die angegebenen Indikationen liegt zwischen 1 und 1000 mg täglich.

Die 13-Alkyl-11β-phenylgonane der allgemeinen Formel I, in denen der 11β-Phenylrest in der 4-Position die erfindungsgemäßen Substituenten trägt, werden nach dem Verfahren gemäß Anspruch 11 hergestellt.

Dabei werden eine Verbindung der allgemeinen Formel II

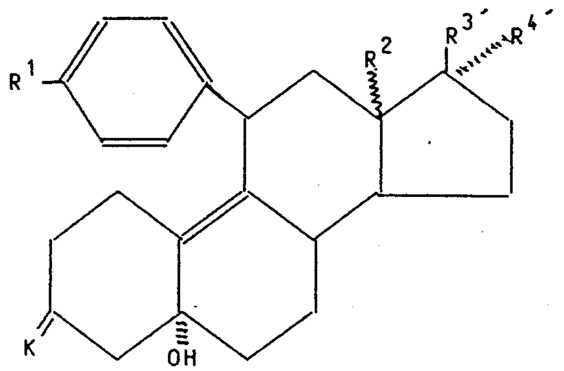

(II) ,

worin K eine in Form des Ketals, des Thioketals, des Oxims oder des Methyloxims blockierte Ketogruppe bedeutet, $R^1$ und $R^2$ die oben genannte Bedeutung haben und $R^{3'}$ und $R^{4'}$ die gleiche Bedeutung wie $R^3$ bzw. $R^4$ haben, wobei in $R^3$ gegebenenfalls vorhandene Hydroxygruppen und in $R^4$ gegebenenfalls vorhandene Hydroxy- und /oder Acylgruppen geschützt sind,

der Einwirkung eines Dehydratisierungsmittels, das auch zur Freisetzung der geschützten Funktion(en) befähigt ist, zur Wasserabspaltung unter Ausbildung der 4(5)-Doppelbindung unterworfen, in Cycloalkyl-, Cycloalkenyl oder Arylrest I c gegebenenfalls vorhandene Hydroxy- und /oder Alkoxyreste gewünschtenfalls erneut geschützt, im Rest I c gegebenenfalls vorhandene Alkylthio- und /oder Dialkylaminoreste gewünschtenfalls zum entsprechenden Sulfoxid, Sulfon und /oder N-Oxid oxidiert und gewünschtenfalls anschließend mit Hydroxylamin-hydrochlorid in Gegenwart von tertiären Aminen bei Temperaturen zwischen -20 °C und +40 °C umgesetzt.

Die saure Behandlung erfolgt in an sich bekannter Weise, indem man die Verbindung der Formel II, die zumindest zwei Schutzgruppen enthält, in einem mit Wasser mischbaren Lösungsmittel, wie wäßrigem Methanol, Ethanol oder Aceton, löst und auf die Lösung katalytische Mengen Mineral- oder Sulfonsäure, wie Salzsäure, Schwefelsäure, Phosphorsäure, Perchlorsäure oder p-Toluolsulfonsäure, oder eine organische Säure, wie Essigsäure, so lange einwirken läßt, bis Wasser abgespalten ist und Schutzgruppen entfernt sind. Die Umsetzung, die bei Temperaturen von 0 bis 100 °C abläuft, kann auch mit einem sauren Ionenaustauscher vorgenommen werden. Der Verlauf der Umsetzung kann mit analytischen Methoden, beispielsweise durch Dünnschichtchromatographie entnommener Proben, verfolgt werden.

Zur Herstellung der Verbindungen der allgemeinen Formel II bestehen gemäß vorliegender Erfindung verschiedene Möglichkeiten.

Bei einer Variante wird ausgehend von einer Verbindung der allgemeinen Formel III

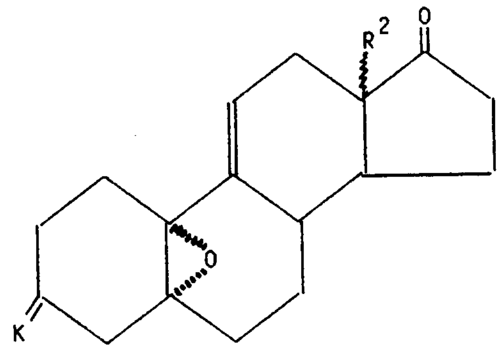

(III) ,

diese nach den üblichen Verfahren des $C_{17}$-Seitenkettenaufbaus durch Einführung der Substituenten $R^3$ und $R^4$ durch nucleophile Addition an das $C_{17}$-Keton und Folgereaktionen ("Terpenoids and Steroids", Specialist Periodical Report, The Chemical Society, London, Vol. 1 - 12) in eine Verbindung der allgemeinen Formel IV überführt

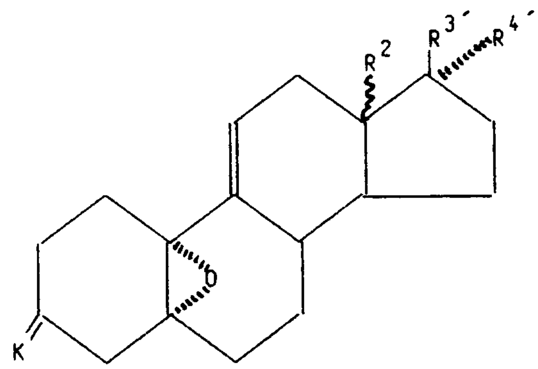

(IV) ,

Der Zugang zur 13α-Methyl-bzw. 13α-Ethylreihe ($R_2$ ist α-ständig) gelingt - wie z.B. in der Europäischen Patentanmeldung 0259 248 beschreiben - durch Bestrahlung von Zwischenprodukten der allgemeinen Formel III (Tetrahedron Letters 26, 2069 (1985), $R_2$ ist β-ständig) mit ultravioletten Licht.

Während die nucleophile Addition an das 17-Keton der 13β-Alkylreihe nur Addukte mit der Hydroxygruppe in β- und der eintretenden Gruppe in α-Stellung zum Funfring liefert, verläuft die Addition an das entsprechende 13-Epi-17-Keton im allgemeinen unter Bildung beider möglicher, isomerer Formen an C-17, die jedoch durch Chromatographie oder fraktionierte Kristallisation leicht trennbar sind. In vielen Fällen sind beide Isomere pharmakologisch wirksam, wenn auch Unterschiede in der Wirkungsstärke bestehen können.

Die nucleophile Addition von HC≡CX, in der X Wasserstoff, Alkyl mit 1 - 4 C- Atomen oder Halogen bedeutet, erfolgt mit Hilfe einer Verbindung der allgemeinen Formel MC≡CX, in der X die oben angegebene Bedeutung hat und M ein Alkalimetall darstellt.

Die metallorganische Verbindung kann auch in situ gebildet und mit dem 17- Keton zur Reaktion gebracht werden. So kann man zum Beispiel auf das 17- Keton in einem geeigneten Lösungsmittel Acetylen und ein Alkalimetall, insbesondere Kalium, Natrium oder Lithium, in Gegenwart eines Alkohols oder in Gegenwart von Ammoniak einwirken lassen. Das Alkalimetall kann auch in Form von zum Beispiel Methyl- oder Butyllithium zur Einwirkung kommen. Als Lösungsmittel sind insbesondedre Dialkylether, Tetrahydrofuran, Dioxan, Benzol und Toluol geeignet.

Zur Herstellung der 17-Chlorethinylverbindung wird die metallorganische Chlorethinylverbindung in situ aus 1,1-Dichlorethylen und einer etherischen Alkalimetall-Lösung, wie zum Beispiel Methyl- oder Butyllithiumlösung, gebildet und mit dem 17-Keton in Lösungsmitteln, wie Tetrahydrofuran oder Diethylether, umgesetzt.

Die 17-Ethinyl-17-hydroxy-Verbindungen lassen sich in alkoholischer Lösung unter Quecksilbersalzkatalyse hydratisieren zu den 17-Acetyl-17-hydroxy-Verbindungen (Chem. Ber. 111, (1978) 3086-3093).

Die Einführung von 3-Hydroxypropin, -progen bzw. -propan in 17-Stellung erfolgt durch Umsetzung des 17-Ketons mit metallierten Derivaten des Propargylalkohols, zum Beispiel mit 1-Lithium-3-tetrahydropyran-2'-yloxy-propin-1, zu den 17-(3-Hydroxy-1-propinyl)-17-hydroxy-Verbindungen, die anschließend zu den 17-(3-Hydroxypropyl- bzw. 3-Hydroxypropenyl)-17-hydroxy-Verbindungen hydriert werden können. Die Hydrierung muß unter Bedingungen durchgeführt werden, die ausschließlich den Angriff an der C-C-Dreifachbindung gewährleisten, ohne die gegebenenfalls vorhandene tetrasubstituierte 9(10)-Doppelbindung abzusättigen. Das gelingt zum Beispiel bei der Hydrierung bei Raumtemperatur und Normaldruck in Lösungsmitteln wie Methanol, Ethanol, Propanol, Tetrahydrofuran (THF) oder Essigester unter Zusatz von Edelmetall-Katalysatoren wie Platin oder Palladium.

Die Einführung der homologen Hydroxyalkin-, Hydroxyalken- und Hydroxyalkangruppen erfolgt in entsprechender Weise mit Homologen des Propargylalkohols.

Die Verbindung mit der Z-konfigurierten Doppelbindung in der Hydroxypropenylgruppe entsteht durch Hydrieren der acetylenischen Dreifachbindung mit einem desaktivierten Edelmetallkatalysator (J. Fried, J.A. Edwards: Organic Reactions in Steroid Chemistry, Van Nostrand Reinhold Company 1972, Seite 134, und H.O. House: Modern Synthetic Reactions 1972, Seite 19). Als desaktivierte Edelmetallkatalysatoren kommen beispielsweise 10 % Palladium auf Bariumsulfat in Gegenwart eines Amins oder 5 % Palladium auf Calciumcarbonat unter Zusatz von Blei(II)-acetat infrage. Die Hydrierung wird nach der Aufnahme von einem Äquivalent Wasserstoff abgebrochen.

Die Verbindung mit der E-konfigurierten Doppelbindung in der Hydroxypropenylgruppe entsteht durch Reduktion der acetylenischen Dreifachbindung in an sich bekannter Weise. In der Literatur sind eine ganze Reihe von Methoden zur Umwandlung von Alkinen in trans-Olefine beschrieben, beispielsweise die Reduktion mit Natrium in flüssigem Ammoniak (J. Am. Chem. Soc. 63 (1941) 216), mit Natriumamid in flüssigem Ammoniak (J. Chem. Soc. 1955, 3558), mit Lithium in niedermolekularen Aminen (J. Am. Chem. Soc. 77 (1955) 3378), mit Boranen (J. Am. Chem. Soc. 93 (1971) 3395 und 94 (1971) 6560), mit Diisobutylaluminiumhydrid und Methyl-Lithium (J. Am. Chem. Soc. 89 (1967) 5085) und insbesondere mit Lithiumaluminiumhydrid/Alkoholat (J. Am. Chem. Soc. 89 (1967) 4245). Eine weitere Möglichkeit ist die Reduktion der Dreifachbindung mit Chrom(II)-sulfat in Gegenwart von Wasser oder Dimethylformamid in schwach saurem Milieu (J. Am. Chem. Soc. 86 (1964) 4358) sowie allgemein die Reduktion durch Einwirkung von Übergangsmetallverbindungen unter Wechsel der Oxydationsstufe.

Werden Endprodukte der Formel I gewünscht mit $R_3/R_4$ in der Bedeutung von

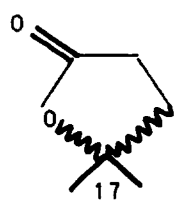

,

so wird die 17-(3-Hydroxypropyl)-Verbindung in an sich bekannter Weise oxydiert, zum Beispiel mit Jones Reagenz, Braunstein, Pyridiniumdichromat, Pyridiniumchlorochromat, Chromsäure-Pyridin oder dem Fetizon-Reagenz Silbercarbonat/Celite (Compt. rend. 267 (1968) 900).

Zur Einführung der Gruppierung

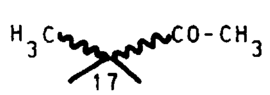

wird das 17-Keton mit Tosylmethylisocyanid in die 17-Nitrilverbindung überführt, aus der 17-Nitrilverbindung wird mit Methyllithium oder Methylmagnesiumbromid die 17-Acetylverbindung erhalten, welche nach Enolisierung mit K-tert.-Butylat in Tetrahydrofuran und Umsetzung mit Methyljodid die gewünschte Gruppierung in 17-Stellung liefert.

Der Aufbau der 17-Cyanmethylseitenkette erfolgt in an sich bekannter Weise aus dem 17-Keton zum Beispiel über das 17-Spiroepoxid und Spaltung des Spiroepoxids mit HCN gemäß Z. Chem. 18 (1978) 259 - 260.

Auch die Einführung der 17-Hydroxyacetylseitenkette erfolgt nach an sich bekannten Methoden, beispielsweise nach der in J. Org. Chem. 47 (1982), 2993 - 2995, beschriebenen Methode.

Freie Hydroxygruppen in 17-Stellung können in an sich bekannter Weise verestert oder veräthert werden.

Die Verbindungen der allgemeinen Formel IV werden anschließend durch Grignard-Addition einer Aryl-Grignard-Verbindung, die in 4-Position bereits den gewünschten Substituenten $R^1$ trägt, zu einer Verbindung der allgemeinen Formel II umgesetzt, die in bereits angegebener Weise weiterverarbeitet wird.

Es ist erfindungsgemäß aber auch möglich, eine Verbindung der allgemeinen Formel IV mit einer Verbindung des Typs

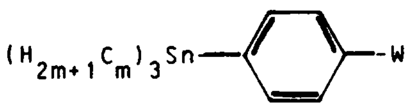 ,

worin W = MgX (X = Br, I) oder vorzugsweise Li (S. H. Lee, R. N. Hanson und D. E. Seitz. Tetrahedron Letters 25, 1751 (1984)) und m = 1, 2, 3, 4, vorzugsweise 1 oder 4 bedeuten,
unter Erzeugung einer Verbindung der allgemeinen Formel V

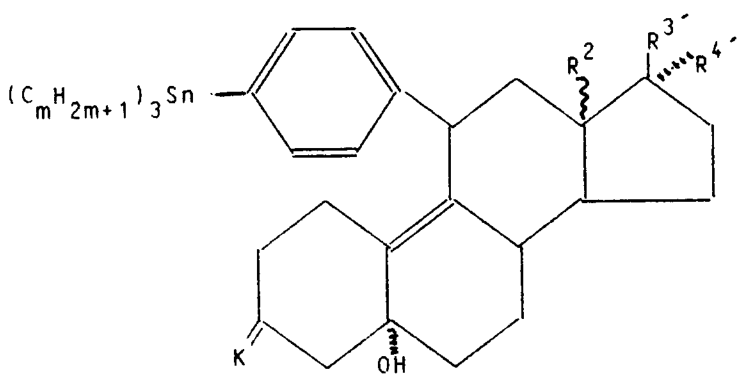

(V) ,

worin K, $R^2$, $R^{3'}$ und $R^{4'}$ die bereits angegebene Bedeutung haben (K. Torssell, J. Goldman. T.E. Petersen; Liebigs Ann. Chem 1973, 231-240) reagieren zu lassen.

Aus einer derartigen Verbindung wird in einer Pd(O)-katalysierten Reaktion in Gegenwart einer Verbindung $R^1Br$, worin $R^1$ der im Endprodukt gewünschte Substituent $R^1$ ist, die Verbindung der allgemeinen Formel II gewonnen.

Soll eine Verbindung der allgemeinen Formel II hergestellt werden, in welcher der Substituent $R^2$ ausschließlich $\alpha$-ständig ist, kann man auch so vorgehen, daß eine Verbindung der allgemeinen Formel IV ($R^2$ = $\alpha$-ständig) mit einem Grignard-Addukt der Formel

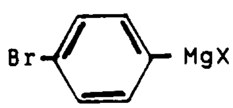

(X = Br, I) zur Reaktion gebracht wird und das Reaktionsprodukt der allgemeinen Formel VI

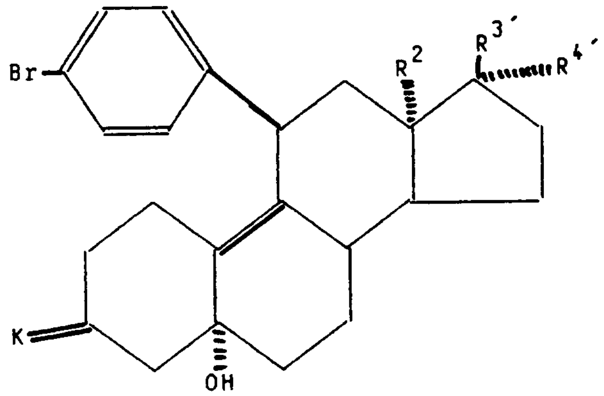

(VI) ,

worin K, $R^2$, $R^{3'}$ und $R^{4'}$ die oben angegebene Bedeutung haben, zur Einführung des $R^1$-Substituenten in der 4-Position des 11$\beta$-konfigurierten Phenylringes in Gegenwart katalytischer Mengen von Pd(O) mit einer zinnorganischen Verbindung der allgemeinen Formel $R^1Sn(C_mH_{2m+1})_3$ (J.K. Stille, Angew. Chem. 98, (1986), 504-519) zur entsprechenden Verbindung der allgemeinen Formel II

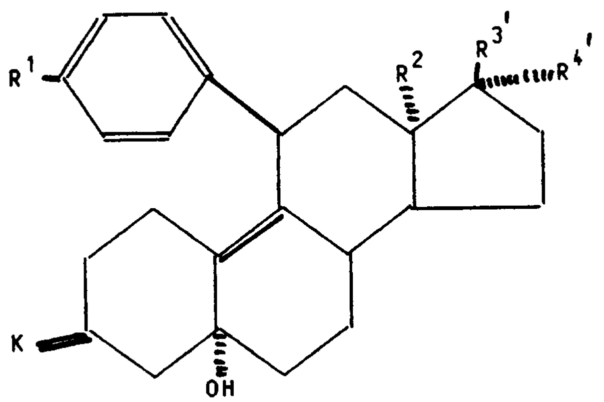

(II)

weiterumgesetzt wird.

Anstelle der zuletzt genannten Variante ist es auch noch möglich, eine Verbindung der allgemeinen Formel VI zunächst Palladium(O)-katalysiert mit einem Hexaalkyldistannan $[(C_mH_{2m+1})_3Sn]_2$ (m = 1, 2, 3, 4, vorzugsweise 1 oder 4) zu einer Verbindung der allgemeinen Formel V umzusetzen und diese - wie bereits angegeben - zu einer Verbindung der allgemeinen Formel II weiterzuverarbeiten (Y. Yamamoto, Y. Azuma, H. Mitoh, Communications, S. 564-565, 1986; T.J. Bailey, Tet. Let. 27, S. 4407-4410, 1986).

Die gemäß Anspruch 12 im Verlauf der möglichen Syntheserouten zu den Ausgangsprodukten der allgemeinen Formel II auftretenden Zwischenprodukte der allgemeinen Formeln III bis VI können alle in Substanz isoliert werden und zählen ebenfalls zum Gegenstand der vorliegenden Erfindung.

Allen vorstehend beschriebenen Verfahrensvarianten ist gemeinsam, daß in die C17-Ketoverbindung der allgemeinen Formel III zuerst durch nucleophile Addition die Vorläufer $R^{3'}$ und $R^{4'}$ des $R^3$ - und $R^4$-Substituenten oder diese beiden Substituenten selbst eingeführt werden unter Bildung einer Verbindung der allgemeinen Formel IV und daß erst danach der 11$\beta$-Phenylrest mit dem entsprechenden Substitutionsmuster in der 4-Position etabliert wird.

Im Gegensatz dazu kann erfindungsgemäß die Synthese der Verbindungen der allgemeinen Formel I auch mit einem 13$\beta$-Alkyl-5,10-epoxid der allgemeinen Formel VII

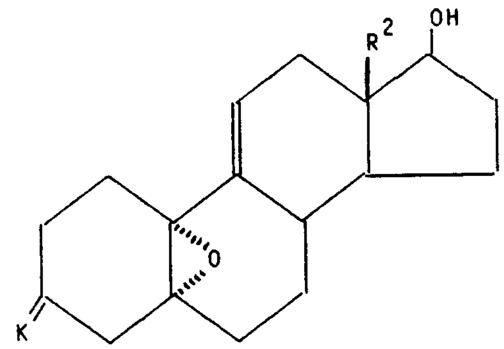

(VII) ,

worin K und $R^2$ die weiter vorne angegebene Bedeutung haben, begonnen werden, indem in dieser zuerst ein in der 4-Position die labile Zinntrialkylgruppe (Alkyl = $C_1$-$C_4$, vorzugsweise $C_1$ oder $C_4$) aufweisender Phenylrest eingeführt wird, durch Grignard-Addition eines geeigneten 4-(Trialkylstannyl)-aryl-Grignard-Verbindung oder Alkylierung mit einer 4-(Trialkylstannyl)-aryllithium-Verbindung unter Bildung einer Verbindung der allgemeinen Formel VIII

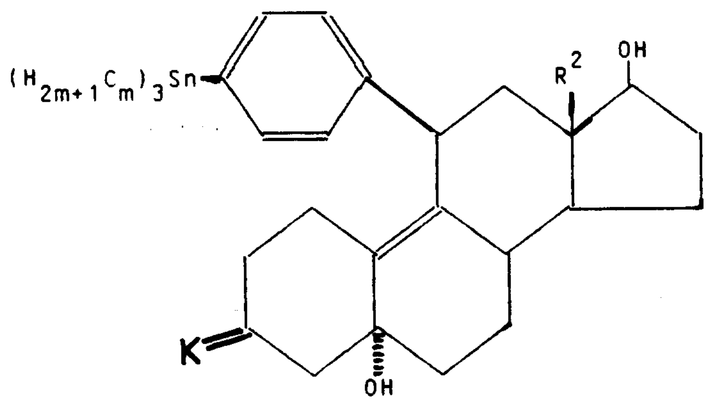

(VIII).

Von einer Verbindung der allgemeinen Formel VIII gelangt man durch übergangsmetallkatalysierte, vorzugsweise Pd(O)-katalysierte Kupplung mit einer Verbindung $R^1$-Y (Y = Br, I) zu einer Verbindung der allgemeinen Formel IX

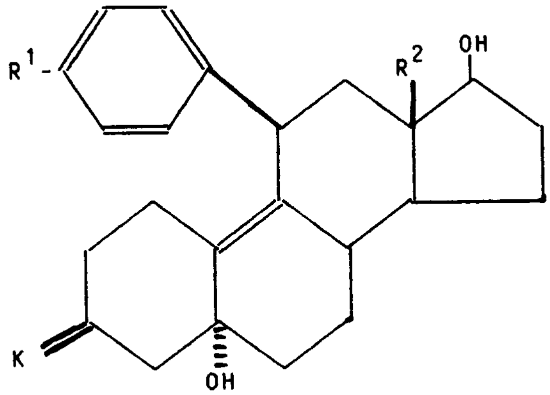

(IX) ,

anschließend durch Oxidation der 17-$\beta$-OH-Funktion und gegebenenfalls nachfolgende Bestrahlung mit ultraviolettem Licht (Umwandlung der 13$\beta$-Alkyl- in eine 13$\alpha$-Alkylgruppe) zu einer Verbindung der allgemeinen Formel X

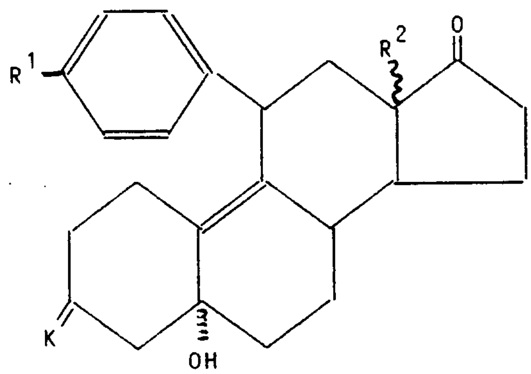

(X) ,

worin die Substituenten die bereits angegebenen Bedeutungen haben und $R^2$ sowohl $\alpha$- als auch $\beta$-ständig sein kann, in die dann in der bereits beschriebenen Art und Weise durch nucleophile Addition der $R^3$ - und $R^4$-Substituent eingeführt und aus der vorhandene Schutzgruppen durch saure Behandlung unter Freisetzung einer erfindungsgemäßen Verbindung der allgemeinen Formel I abgespalten werden.

Die Reihenfolge der hier beschriebenen Reaktionsschritte, verlaufend über die Verbindungen der Formeln IX und X, ausgehend von einer Verbindung der Formel VIII, kann, ausgehend von derselben Verbindung VIII auch vertauscht werden, indem zuerst die 17$\beta$-OH- zur entsprechenden 17-Keto-Funktion oxidiert, anschließend gegebenenfalls mit ultraviolettem Licht bestrahlt und dann durch übergangsmetall-, vorzugsweise Pd(O)-katalysierte Kupplung mit einer Verbindung $R^1$-Y (Y = Br, I) die bereits vorstehend beschriebene Zwischenverbindung der allgemeinen Formel X erzeugt wird, welche dann noch - wie ebenfalls bereits beschrieben - zu einer Verbindung der Formel I umgesetzt wird. Dabei wird eine Verbindung der allgemeinen Formel XI durchlaufen:

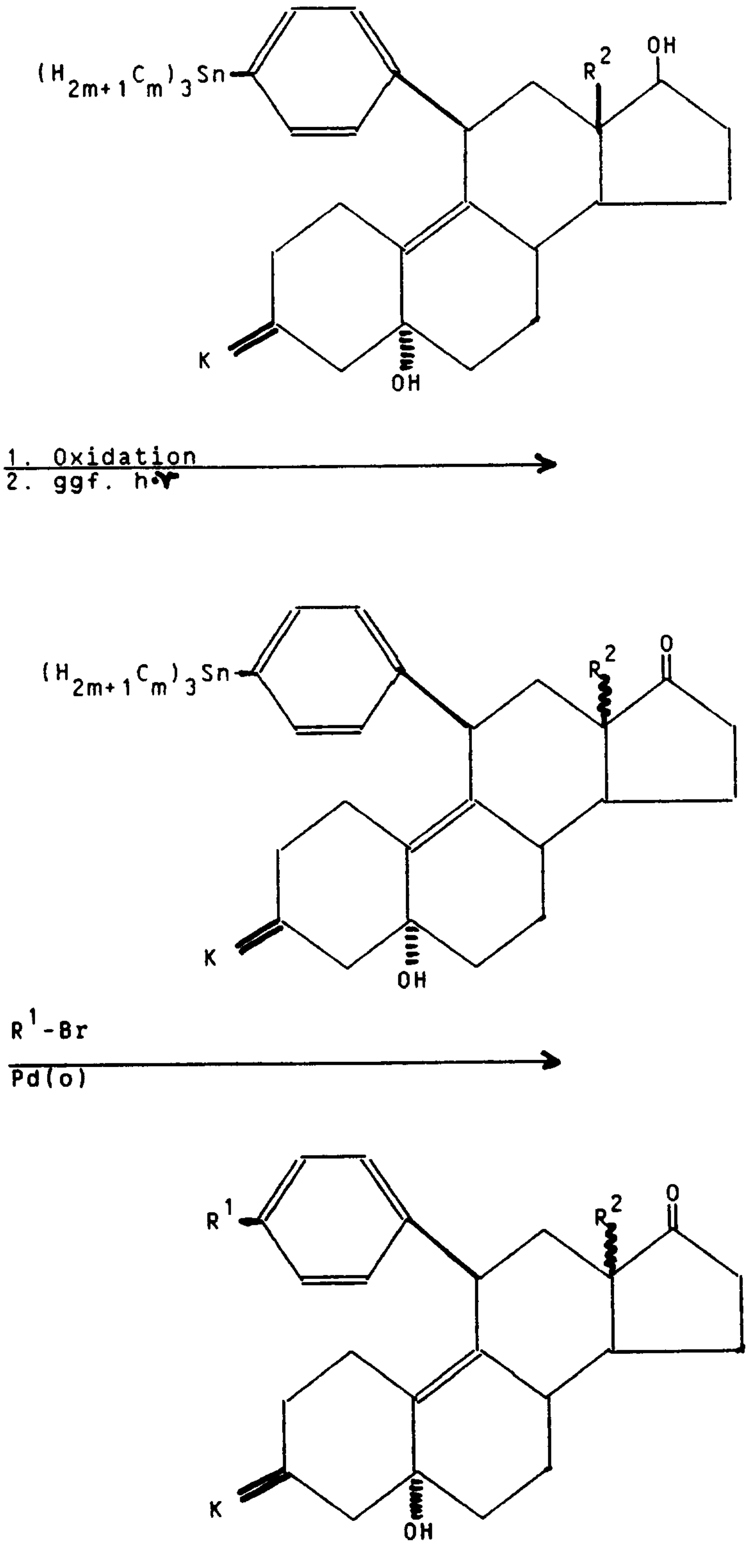


(VIII)

(XI)

(X) .

Bei einer Verbindung der allgemeinen Formel (XI) kann auch zuerst an das C-17-Atom nucleophil addiert werden zur Etablierung der Reste $R^3$ und $R^4$ bzw. deren Vorläufer $R^{3'}$ und $R^{4'}$ unter Bildung einer Verbindung der allgemeinen Formel V, die dann, wie schon angegeben, weiterzuverarbeiten ist.

Die Verbindungen der allgemeinen Formeln VIII bis XI können ebenso wie diejenigen der Formeln III bis VI in Substanz isoliert werden und gehören zum Gegenstand vorliegender Erfindung.

Die nachfolgenden stellvertretenden Beispiele dienen der Erläuterung der Erfindung.

Allgemeine Vorschrift zur Herstellung der Verbindungen der allgemeinen Formel I durch saure Spaltung der Verbindungen der allgemeinen Formel II

**(Tabelle 2)**

Eine Lösung von x g Steroid der allgemeinen Formel II in y ml 70%iger Essigsäure wird z Minuten bei t °C gerührt. Anschließend gießt man das Reaktionsgemisch auf Eiswasser, neutralisiert durch Zugabe von wäßriger Ammoniak-Lösung und extrahiert mit Dichlormethan. Die vereinigten organischen Phasen werden mit gesättigter Kochsalzlösung gewaschen, über Natriumsulfat getrocknet und im Vakuum eingeengt. Durch Chromatographie an Kieselgel mit einem Gemisch aus Ethylacetat/Hexan erhält man aus dem Rohprodukt a g der gewünschten Verbindung der allgemeinen Formel I.

Allgemeine Vorschrift zur Herstellung der Verbindungen der allgemeinen Formel II

A) durch Grignard-Addition

**(Tabelle 3)**

10,8 mmol Magnesiumspäne werden unter Schutzgas in 5 ml absolutem Tetrahydrofuran vorgelegt und mit 0,8 mmol Dibromethan versetzt. Nach erfolgter Reaktion wird eine Lösung von 10 mmol Halogenaromat in 11 ml absolutem Tetrahydrofuran langsam zugetropft. Nach vollständiger Umsetzung wird die Grignard-Lösung auf 0 °C abgekühlt und mit 0,29 mg Kupfer-(I)-chlorid versetzt. Anschließend wird eine Lösung von 12 mmol Epoxid EP in 7,5 ml absolutem Tetrahydrofuran zugetropft. Das Reaktionsgemisch wird über Nacht langsam auf Raumtemperatur erwärmt und dann auf gesättigte Ammoniumchloridlösung gegossen. Man extrahiert die wäßrige Phase mit Ethylacetat, vereinigt die organischen Phasen, wäscht sie mit gesättigter Kochsalzlösung und trocknet sie über Natriumsulfat. Nach Einengen am Vakuum wird der Rückstand an Aluminumoxid (Merck, Stufe III, neutral) mit einem Gemisch aus Ethylacetat/Hexan chromatographiert. Es werden y mmol des gewünschten Addukts Y als weißer Schaum isoliert.

B) durch Kupplung von Steroidstannanen der allgemeinen Formel V mit Arylbromiden

**Tabelle 4**

In 60 ml absolutem Dioxan werden unter Schutzgas 2,27 mmol Stannylsteroid vorgelegt, nacheinander mit 27,1 mmol Arylbromid sowie 0,228 mmol Bis- (triphenylphosphin)-palladium(II)chlorid versetzt und 24 Stunden unter Rückfluß erhitzt. Die Reaktionsmischung wird über Celite filtriert, mit Essigester nachgewaschen und mit dem gleichen Volumen einer 10%igen Ammoniaklösung versetzt. Nach 30-minütigem Rühren wird die organische Phase abgetrennt, mit gesättigter NaCl-Lösung gewaschen und getrocknet. Nach Einengen im Vakuum wird der Rückstand an Aluminiumoxid (Merck, Stufe III, neutral) mit einem Gemisch aus Ethylacetat/Hexan chromatographiert. Man erhalt a g der gewünschten Verbindung der allgemeinen Formel II (Tabelle 4).

C) durch Kupplung von Bromsteroiden der allgemeinen Formel VI mit Heteroarylstannanen (Darstellung analog Lit.: Organometal. Chem., 246 (1983) 163)

**Tabelle 5**

In 50 ml absolutem Dioxan werden unter Schutzgas 2,23 mmol Bromsteroid vorgelegt, nacheinander mit 22,3 mmol Arylstannan sowie 0,228 mmol Bis-(triphenylphosphin)-palladium(II)chlorid versetzt und 2 Stunden unter Rückfluß erhitzt. Nach der unter a) beschriebenen Aufarbeitung werden b g der gewünschten Verbindung der allgemeinen Formel II erhalten (Tabelle 5).

**Beispiel 1**

**17-(Prop-1-inyl)-17β-hydroxy-11β-(4-vinylphenyl)-4,9-estradien-3-on**

**(Tabelle 2)**

a) Herstellung des Ausgangssubstrates für die Grignard-Addition
17-(Prop-1-inyl)-5α, 10α-epoxy-3,3-(2,2-dimethyltrimethylendioxy)-9(11)-estren-17β-ol
1,3 l absolutes Tetrahydrofuran werden bei 0°C durch Einleiten von Propin mit diesem Gas gesättigt. Anschließend wird die Lösung auf -10°C heruntergekühlt und langsam mit 166 ml einer 1.6 m Losung von n-Butyllithium in Hexan versetzt. Nach 15 minutigem Nachrühren bei 0°C wird langsam eine Lösung von 9,9 g 5α, 10α-Epoxy-3,3-(2,2-dimethyltrimethylendioxy)-9(11)-estren-17-on in 250 ml absolutem Tetrahydrofuran zugetropft. Nach Zugabe wird das Reaktionsgemisch für weitere 30 Minuten bei 0°C gerührt und dann auf Eiswasser gegossen. Die wäßrige Phase extrahiert man mit Ethylacetat, vereinigt die organische Phase und wäscht sie mit gesättigter Kochsalzlösung. Nach Trocknen über Natriumsulfat und Einengen im Vakuum chromatographiert man den so erhaltenen Rückstand an Aluminiumoxid (Merck, Stufe III, neutral) mit einem Gemisch aus Ethylacetat/Hexan. Es werden 10,2 g 17-(Prop-1-inyl)-5α, 10α-epoxy-3,3-(2,2-dimethyltrimethylendioxy)-9(11)-estren-17β-ol als weißer Schaum isoliert.
IR (KBr) : 2245 $cm^{-1}$ C-C- Dreifachbindung.
b) 17-(Prop-1-inyl)-11β-(4-vinylphenyl)-3,3-(2,2-dimethyltrimethylendioxy)-9-estren-5α, 17β-diol
(Tabelle 3)

**Beispiel 2**

**17-(3-Hydroxyprop-1-(Z)-enyl]-17β-hydroxy-11β-(4-vinylphenyl)-4,9-estradien-3-on**

a) Herstellung des Ausgangssubstrates für die Grignard-Addition
17-[3-Tetrahydropyran-2-yloxy)-prop-1-(Z)-enyl]-5α, 10α-epoxy-3,3-(2,2-dimethyltrimethylendioxy-9(11)-estren-17β-ol
20 g 17-[3-(Tetrahydropyran-2-yloxy)-prop-1-inyl]-5α,10α-epoxy-3,3-(2,2-dimethyltrimethylendioxy)-9(11)-estren-17β-ol werden in 400 ml Ethanol gelost und mit 40 ml Pyridin und 4 g Palladium/Barimsulfat (10%ig) versetzt. Anschließend wird bei Normaldruck mit Wasserstoff hydriert. Nach Aufnahme eines Equivalents Wasserstoff wird der Katalysator durch Filtration über Celite abgetrennt, das Filtrat eingeengt und der Rückstand an Aluminumoxid (Merck, Stufe III, neutral) mit einem Gemisch aus Ethylacetat/Hexan chromatographiert. Es werden 16,8 g 17-[3-(Tetrahydropyran-2-yloxy)-prop-1-(Z)-enyl]-5α, 10α-epoxy-3,3-(2,2-dimethyltrimethylendioxy)-9(11)-estren17β-ol als weißer Schaum isoliert.
b) 17-[3-(Tetrahydropyran-2-yloxy)-prop-1-(Z)-enyl]-11β-(4-vinylphenyl)-3,3-(2,2-dimethyltrimethylendioxy]-9-estren-5α,17β-diol
(Tabelle 3)

**Beispiel 3**

**17-(3-Hydroxypropyl)-17α-hyrdoxy-13α-methyl-11β-(4-isopropenylphenyl)-4,9-gonadien-3-on**

a) Herstellung des Substrats für die Grignard-Addition
17-[3-(Tetrahydropyran-2-yloxy)-propyl]-13α-methyl-5α, 10α-epoxy-3,3-(2,2-dimethyltrimethylendioxy)-9-(11)-gonen-17α-ol
5.1 g 17-[3-(Tetrahydropyran2-yloxy)-prop-1-inyl]-13α-methyl-5α, 10α-epoxy-3,3-(2,2-dimethyltrimethylendioxy)-9(11)-gonen-17α-ol werden in 150 ml Tetrahydrofuran gelöst und mit 2,55 g Tris-(triphenylphosphin)-rhodium-(I)-chlorid versetzt. Danach wird bei Normaldruck 20 Stunden mit Wasserstoff hydriert. Anschließend wird das Reaktionsgemisch eingeengt und der Rückstand an Aluminiumoxid (Merck, Stufe III, neutral) mit einem Gemisch aus Ethylacetat/Hexan chromatographiert. Es werden 4,9 g der gewünschen Verbindung als weißer Schaum isoliert.
b) 17-[3-(Tetrahydropyran-1-yloxy)-propyl]-13α-methyl-11β-(4-isopropenylphenyl)-3,3-(2,2-dimethyltrimethylendioxy)-9-gonen-5α, 17α-diol
(Tabelle 3)

**Beispiel 4**

**17-(3-Hydroxypropyl)-17α-hydroxy-13α-methyl-11β-(4-ethylphenyl)-4,9-gonadien-3-on**

a) Als Ausgangsprodukt für die Grignard-Addition dient das in Beispiel 3a beschriebene Substrat.
b) 17-[3-(Tetrahydropyran-2-yloxy)-propyl]-13α-methyl-11β-(4-ethylphenyl)-3,3-(2,2-dimethyltrimethylendioxy)-9-gonen-5α, 17α-diol
(Tabelle 3)

**Beispiel 5**

**17-(3-Hydroxypropyl)-17α-hydroxy-13α-methyl-11β-(4-vinylphenyl)-4,9-gonadien-3-on**

a) Als Ausgangsproprodukt für die Grignard-Addition dient ebenfalls das in Beispiel 3a beschriebene Substrat.
b) 17-[3-(Tetrahydropyran-2-yloxy)-propyl]-13α-methyl-11β-(4-vinylphenyl)-3,3-(2,2-dimethyltrimethylendioxy)-9-gonen-5α, 17α-diol
(Tabelle 3)

Beispiel 6 (vgl. Beispiel 8)

17-Hydroxy-17-(3-hydroxy-(Z)-prop-1-enyl)-11β-[4-(3-thienyl)-phenyl] -4,9-estradien-3-on (Tabelle 2)

a)Als Ausgangsprodukt für die Grignard-Addition mit 1-Chlor-4-(3-thienyl)-benzol (Darstellung analog Lit.: Tetrahedron Letters 27, 4407 (1986) dient das in Beispiel 2a beschriebene Substrat.
b)3,3-(2,2-Dimethyltrimethylendioxy)-17-(3-(tetrahydropyran-2-yloxy)-prop-(Z)-1-enyl]-11β-[4-(3-thienyl)-phenyl]-estr-9-en-5α,17β-diol (Tabelle 3)

Beispiel 7

17-Hydroxy-17-(3-hydroxyprop-(Z)-1-enyl)-11β-[4-(3-pyridyl)-phenyl]-4,9-estradien-3-on (Tabelle 2)

a)3,3-(2,2-Dimethyltrimethylendioxy)-17-[3-(tetrahydropyran-2-yloxy)-prop-(Z)-1-enyl]-11β-(4-tri-n-butylstannylphenyl)-estr-9-en-5α,17β-diol
30.6g 1,4-Bis-tri-n-butylzinnbenzol [Darstellung analog Lit.: Chem.Ber.87, 1255(1954)] werden unter Schutzgas in 120 ml absolutem Tetrahydrofuran bei -78° C vorgelegt und mit 29ml einer 1.6M Lösung von n-Butyllithium in Hexan tropfenweise versetzt. Es wird 2 Stunden bei -78°C nachgerührt und mit 2.0g Kupfer(I)cyanid versetzt. Nach 30 Minuten wird eine Lösung von 4.0g der unter Beispiel 2abeschriebenen Verbindung in 10ml absolutem Tetrahydrofuranzugetropft. Nach Zugabe wird das gemisch auf Raumtemperatur erwärmt,48 Stunden nachgerührt und dann auf Eiswasser gegossen. Die wäßrigePhase extrahiert man mit Essigester, vereinigt die organischen Phasen und wäscht sie mit gesättigter Kochsalzlösung. Nach Trocknen über Natriumsulfat und Einengen am Vakuum chromatographiert man den so erhaltenen Rückstand an Kieselgel mit einem Gemisch aus Essigester/Hexan/Triethylamin. Es werden 3.5g der obigen Verbindung erhalten.
1H-NMR(CDCl3 + Py*d5) $\delta$ = 0.50(s,3H,18-H), 0.86(s,3H,Acetal-Me), 0.88,0.90,0.92(3t,9H,Me), 1.05-(s,3H,Acetal-Me), 3.40-3.59(m,4H,Acetal-CH2), 4.26(d,J = 7Hz,1H,11α -H), 4.75(m,1H,H-THP-ether), 5.52-5.82(m,2H,H-olefin.,C-20 und C-21), 7.15,7.30(AA'BB'-System,J = 9Hz,4H,Ar-H).
b)3,3-(2,2-Dimethyltrimethylendioxy)-11β-[4-(3-pyridyl)-phenyl]-17-[3-(tetrahydropyran-2-yloxy)-prop-(Z)-1-enyl]-estr-9-en-5α,17β-diol (Tabelle 4).

Beispiel 8 (vgl. Beispiel 6)

17-Hydroxy-17α-(3-hydroxy-(Z)-prop-1-enyl)-11β-[4-(3-thienyl)-phenyl]-4,9-estradien-3-on (Tabelle 2).

a)Als Ausgangsprodukt für die Kupplung nach der allgemeinen Vorschrift a) dient das in Beispiel 7a beschriebene Substrat
b)3,3-(2,2-Dimethyltrimethylendioxy)-17-[3-(tetrahydropyran-2-yloxy)-prop-(Z)-1-enyl]-11β-[4-(3-thienyl)-phenyl]-estr-9-en-5α,17β-diol (Tabelle 4).

Die Verbindung aus Beispiel 6 ist mit der aus Beispiel 8 identisch.

Beispiel 9

17-Hydroxy-17α-(3-hydroxy-(Z)-prop-1-enyl)-11β-[4-(3-furyl)-phenyl]-4,9-estradien-3-on (Tabelle 2).

a)Als Ausgangsprodukt für die Kupplung nach der allgemeinen Vorschrift a dient das in Beispiel 7a beschriebene Substrat.
b)3,3-(2,2-Dimethyltrimethylendioxy)-17-[3-(tetrahydropyran-2-yloxy)-prop-(Z)-1-enyl]-11β-[4-(3-furyl)-phenyl]-estr-9-en-5α,17β-diol (Tabelle 4)

Beispiel 10

17-Hydroxy-17α-(3-hydroxyprop-(Z)-1-enyl)-11β-[4-(2-thiazolyl)-phenyl]-4,9-estradien-3-on (Tabelle 2)

a)Als Ausgangsprodukt für die Kupplung nach der allgemeinen Vorschrift a dient das in Beispiel 7a beschriebene Substrat.
b)3,3-(2,2-Dimethyltrimethylendioxy)-17-[3-(tetrahydropyran-2-yloxy)-prop-(Z)-1-enyl]-11β-[4-(2-thiazolyl)-phenyl]-estr-9-en-5α,17β-diol (Tabelle 4)

Beispiel 11

17-Hydroxy-17β-(3-hydroxypropyl)-13α-methyl-11β-[4-(3-pyridyl)-phenyl]-4,9-gonadien-3-on (Tabelle 2).

a)11β-(4-Bromphenyl)-3,3-(2,2-dimethyltrimethylendioxy)-13α-methyl-17-[3-(tetrahydropyran-2-yloxy)-propyl]-9-gonen-5α,17α-diol
3.16g Magnesiumspäne werden unter Schutzgas in 15 ml absolutem Tetrahydrofuran vorgelegt und mit 0.1ml Dibromethan versetzt. Nach erfolgter Reaktion wird eine Lösung von 30.7g 1,4-Dibrombenzol in 500ml absolutem Tetrahydrofuran langsam zugetropft. Nach vollständiger Umsetzung (30 Minuten bei 40 C) wird die Grignard-Lösung auf 0 C abgekühlt und mit 160mg Kupfer-I-chlorid versetzt. Anschließend wird eine Lösung von 5.6g des in Beispiel 3a beschriebenen Epoxids in 110ml absolutem Tetrahydrofuran zugetropft. Das Reaktionsgemisch wird wie in der allg. Vorschrift beschrieben aufgearbeitet. Es werden nach Säulenchromatographie 6.6g der obigen Verbindung erhalten.
b)3,3-(2,2-Dimethyltrimethylendioxy)-13α-methyl-17-[3-(tetrahydropyra n-2-yloxy)-propyl]-11β-(4-tri-n-butylstannylphenyl)-9-gonen-5α,17α-diol
6.27g der unter a) hergestellten Verbindung werden unter Schutzgas in 180ml absolutem Dioxan vorgelegt, mit 15ml Hexabutyldizinn, 625mg Bis-(triphenylphosphin)-palladium-II-chlorid und 2.5g Tetrabutylammoniumchlorid versetzt und anschließend 2 Stunden unter Rückfluß erhitzt. Nach Filtration über Celite wird am Vakuum eingeengt und der Rückstand an Aluminiumoxid mit einem Gemisch aus Essigester/Hexan chromatographiert. Es werden 4.81g der obigen Verbindung erhalten.
c)3,3-(2,2-Dimethyltrimethylendioxy)-13α-methyl-11β-[4-(3-pyridyl)-phenyl]-17-[3-(tetrahydropyran-2-yloxy)-propyl]-9-gonen-5α,17α-diol (Tabelle 4)

Beispiel 12

17-Hydroxy-17β-(3-hydroxypropyl)-13α-methyl-11β-[4-(4-cyanophenyl)-phenyl]-4,9-gonadien-3-on (Tabelle 2)

a)Als Ausgangsprodukt für die Kupplung nach der allgemeinen Vorschrift a dient das in Beispiel 11b beschriebene Substrat.
b)3,3-(2,2-Dimethyltrimethylendioxy)-13α-methyl-11β-[4-(4-cyanophenyl)-phenyl]-17-[3-(tetrahydropyran-2-yloxy)-propyl]-9-gonen-5α,17α-diol (Tabelle 4)

Beispiel 13

17-Hydroxy-17β-(3-hydroxypropyl)-13α-methyl-11β-[4-(5-pyrimidyl)-phenyl]-4,9-gonadien-3-on (Tabelle 2)

a)Als Ausgangsprodukt für die Kupplung nach der allgemeinen Vorschrift α dient das in Beispiel 11b beschriebene Substrat.
b)3,3-(2,2-Dimethyltrimethylendioxy)-13α-methyl-11β-[4-(5-pyrimidyl)-phenyl]-17-[3-(tetrahydropyran-2-yloxy)-propyl]-9-gonen-5α,17α-diol (Tabelle 4)

Beispiel 14 (vgl. Beispiel 17)

17-Hydroxy-17β-(3-hydroxypropyl)-13α-methyl-11β-[4-(3-thienyl)-phenyl-4,9-gonadien-3-on (Tabelle 2)

a)Als Ausgangsprodukt für die Kupplung nach der allg. Vorschrift b dient das in Beispiel 11a beschriebene Substrat.
b)3,3-(2,2-Dimethyltrimethylendioxy)-13α-methyl-17-[3-(tetrahydropyran-2-yloxy)-propyl]-11β-[4-(3-thienyl)-phenyl]-9-gonen-5α,17α-diol (Tabelle 5)

Beispiel 15

17-Hydroxy-17β-(3-hydroxypropyl)-13α-methyl-11β-[4-(3-furyl)-phenyl]-4,9-gonadien-3-on (Tabelle 2)

a)Als Ausgangsprodukt für die Kupplung nach der allg. Vorschrift b dient das in Beispiel 11a beschriebene Substrat.
b)3,3-(2,2-Dimethyltrimethylendioxy)-13α-methyl-11β-[4-(3-furyl)-phenyl]-17-[3-(tetrahydropyran-2-yloxy)-propyl]-9-gonen-5α,17α-diol (Tabelle 5)

Beispiel 16

17-Hydroxy-17β-(3-hydroxypropyl)-13α-methyl-11β-[4-(2-thiazolyl)-phenyl]-4,9-gonadien-3-on (Tabelle 2)

a)Als Ausgangsprodukt für die Kupplung nach der allgemeinen Vorschrift b dient das in Beispiel 11a beschriebene Substrat.
b)3,3-(2,2-Dimethyltrimethylendioxy)-13α-methyl-11β-[4-(2-thiazolyl)-phenyl]-17-[3-(tetrahydropyran-2-yloxy)-propyl]-9-gonen-5α,17α-diol (Tabelle 5)

Beispiel 17 (vgl. Beispiel 14)

17-Hydroxy-17β-(3-hydroxypropyl)-13α-methyl-11β-[4-(3-thienyl)-phenyl]-4,9-gonadien-3-on (Tabelle 2) Die Verbindung aus Beispiel 14 ist mit der aus Beispiel 17 identisch.

a)3,3-(2,2-Dimethyltrimethylendioxy)-11β-(4-tri-n-butylstannylphenyl)-estr-9-en-5α,17β-diol
Aus 10g 3,3-(2,2-Dimethyltrimethylendioxy)-5α,10α-epoxy-estr-9(11)-en-17β-ol werden, wie unter Beispiel 7a beschrieben, 5.1g der obigen Verbindung erhalten.
1-H-NMR(CDCl3 + Py*d5) δ = 0.38(s,3H,18-H),0.87(s,3H,Acetal-Me), 0.89,0.91,0.93(3t,9H,Me), 1.05-(s,3H,Acetal-Me), 3.42-3.67(m,5H,17-H und Acetal-CH2), 4.22(d,J = 7Hz,1H,11α -H), 4.40(s,breit,1H,OH), 7.17,7.31(AA'BB'-System,J = 9Hz,4H,Ar-H).
b)3,3-(2,2-Dimethyltrimethylendioxy)-11β-[4-(3-thienyl)-phenyl]-estr-9-en-5α,17β-diol
Aus 5.1g der unter 17a beschriebenen Verbindung werden nach der allgemeinen Vorschrift a 2.8g der obigen Verbindung erhalten.
c)3,3-(2,2-Dimethyltrimethylendioxy)-5α-hydroxy-11β-[4-(3-thienyl)-phenyl]-estr-9-en-17-on
Aus 2.8g der unter 17b beschriebenen Verbindung, 1.6g Aluminiumtriisopropylat und 11.6ml Cyclohexanon in 60ml absolutem Toluol werden nach 3 stündigem Erhitzen am Wasserabscheider nach der üblichen Aufarbeitung und säulenchromatographischer Reinigung 2.05g der obigen Verbindung erhalten.
d)3,3-(2,2-Dimethyltrimethylendioxy)-5α-hydroxy-13α-methyl-11β-[4-(3-thienyl)-phenyl]-9-gonen-17-on
Aus 2.05g der unter 17c beschriebenen Verbindung in 600ml absolutem Dioxan werden nach 10 minütiger Bestrahlung bei Raumtemperatur mit einer Quecksilberhochdrucklampe (Philips HPK 125) und chromatographischer Reinigung 840mg der obigen Verbindung erhalten.

e)3,3-(2,2-Dimethyltrimethylendioxy)-13α-methyl-17-[3-(tetrahydropyran-2-yloxy)-1-propinyl]-11β-[4-(3-thienyl-phenyl]-9-gonen-5α,17α-diol

Aus 840mg der unter 17d beschriebenen Verbindung in 20ml absolutem Tetrahydrofuran, 4.52g 2-(Propargyloxy)-tetrahydropyran in 80ml absolutem Tetrahydrofuran und 19ml einer 1.6M Lösung von n-Butyllithium in Hexan werden nach chromatographischer Reinigung 370mg der obigen Verbindung erhalten.

f)3,3-(2,2-Dimethyltrimethylendioxy)-13α-methyl-17-[3-(tetrahydropyra n-2-yloxy)-propyl]-11β-[4-(3-thienyl)-phenyl]-9-gonen-5α,17α-diol

Aus 370mg der unter 17e hergestellten Verbindung werden, wie unter Beispiel 3a beschrieben, 310mg der obigen Verbindung erhalten.

Beispiel 18

3,3-(2,2-Dimethyltrimethylendioxy)-13α-ethyl-5α-hydroxy-11β-[4-(3-thienyl)-phenyl]-9-gonen-17-on

Aus 2.9g 3,3-(2,2-Dimethyltrimethylendioxy)-5α-hydroxy-18-methyl-11β-[4-(3-thienyl)-phenyl]-estr-9-en-17-on (Darstellung analog Beispiel 17) werden wie unter 17d beschrieben nach chromatographischer Reinigung 970mg der obigen Verbindung erhalten.

1H-NMR(CDCl3 + Py-d5) δ = 0.84(s,3H,Acetal-Me), 0.88(t,3H,18-Me), 1.02(s,3H,Acetal-Me), 3.35-3.56-(m,4H,Acetal-CH2), 3.80(m,1H,11α -H), 4.42(s,breit,1H,OH), 7.13,7.50(AA'BB'-System,J = 9Hz,4H,Ar-H), 7.38-7.45(m,3H,Ar-H).

Beispiel 19

17β-Hydroxy-18-methyl-17-(1-propinyl)-11β-[4-(3-thienyl)-phenyl]-est ra-4,9-dien-3-on (Tabelle 2)

1H-NMR(CDCl3) δ = 0.31(t,J = 7Hz,3H,18-Me), 1.91(s,3H,C≡C-Me), 4.45(d,J = 7Hz,1H,11α-H), 5.78(s,1H,4-H), 7.21,7.50(AA'BB'-System,J = 9Hz,4H,Ar-H), 7.39-7.46(m,3H,Ar-H).

a)Als Ausgangsprodukt für die Propinylierung dient das in Beispiel 18 verwendete Startmaterial.

b)3,3-(2,2-Dimethyltrimethylendioxy)-18-methyl-17-(1-propinyl)-11β-[4-(3-thienyl)-phenyl]-estr-9-en-5α,17β-diol

Aus 1.91g 3,3-(2,2-Dimethyltrimethylendioxy)-5α-hydroxy-18-methyl-11β-[4-(3-thienyl)-phenyl]-estr-9-en-17-on in 40ml absolutem Tetrahydrofuran, 180ml einer mit Propin gesättigten Tetrahydrofuranlösung und 24ml einer 1.6M Lösung von Butyllithium in Hexan werden nach chromatographischer Reinigung 1.47g der obigen Verbindung erhalten.

**Tabelle 2**

| Beispiel | Ansatz x [g] | Ansatz y [ml] | Reaktionsparameter t [°C] | Reaktionsparameter z [min] | Ausbeute a [g] | Ausbeute $[\alpha]_D^{20}$ |
|---|---|---|---|---|---|---|
| 1 | 10 | 100 | 50 | 60 | 6,54 | 131 ($CHCl_3$;c=0,53) |
| | Steroid 1b | | | | Steroid Beispiel 1 | |
| 2 | 15 | 150 | 50 | 60 | 6,25 | 218 ($CHCl_3$;c=0,505) |
| | Steroid 2b | | | | Steroid Beispiel 2 | |
| 3 | 5 | 50 | 50 | 180 | 1,73 | 380 ($CHCl_3$;c=0,515) |
| | Steroid 3b | | | | Steroid Beispiel 3 | |
| 4 | 1,4 | 35 | 50 | 60 | 0,68 | 349 ($CHCl_3$;c=0,510) |
| | Steroid 4a | | | | Steroid Beispiel 4 | |
| 5 | 1,1 | 28 | 50 | 60 | 0,41 | 419 ($CHCl_3$;c=0,500) |
| | Steroid 5a | | | | Steroid Beispiel 5 | |
| 6 | 1.4 | 15 | 50 | 60 | 0.54 | 249 c=0.52 |
| | Steroid 6b | | | | Steroid Beispiel 6 | |
| 7 | 0.74 | 10 | 50 | 40 | 0.30 | 208 c=0.505 |
| | Steroid 7b | | | | Steroid Beispiel 7 | |
| 8 | 1.3 | 14 | 50 | 40 | 0.40 | 250 c=0.52 |
| | Steroid 8b | | | | Steroid Beispiel 8 | |
| 9 | 1.2 | 15 | 50 | 45 | 0.53 | 228 c=0.505 |
| | Steroid 9b | | | | Steroid Beispiel 9 | |
| 10 | 1.4 | 18 | 50 | 30 | 0.68 | 297 c=0.505 |
| | Steroid 10b | | | | Steroid Beispiel 10 | |
| 11 | 0.60 | 8 | 50 | 40 | 0.27 | 395 c=0.51 |
| | Steroid 11c | | | | Steroid Beispiel 11 | |
| 12 | 0.86 | 11 | 50 | 45 | 0.35 | 438 c=0.51 |
| | Steroid 12b | | | | Steroid Beispiel 12 | |
| 13 | 0.80 | 12 | 50 | 30 | 0.30 | 390 c=0.51 |
| | Steroid 13b | | | | Steroid Beispiel 13 | |
| 14 | 1.0 | 15 | 50 | 45 | 0.46 | 424 c=0.505 |
| | Steroid 14b | | | | Steroid Beispiel 14 | |
| 15 | 1.3 | 19 | 50 | 45 | 0.50 | 389 c=0.51 |
| | Steroid 15b | | | | Steroid Beispiel 15 | |
| 16 | 0.80 | 9 | 50 | 45 | 0.34 | 431 c=0.5 |
| | Steroid 16b | | | | Steroid Beispiel 16 | |
| 17 | 0.31 | 6 | 30 | 30 | 0.11 | 421 c=0.51 |
| | Steroid 17f | | | | Steroid Beispiel 17 | |
| 19 | 0.32 | 6 | 50 | 30 | 0.14 | -- |
| | Steroid 19b | | | | Steroid Beispiel 19 | |

**Tabelle 3**

| Beispiel | Ansatz Epoxid **EP** | [mmol] | [g] | Halogenaromat (X=Br,I) | Ausbeute Addukt Y | [mmol] | [g] |
|---|---|---|---|---|---|---|---|
| 1 | Steroid 1a | 24 | 9,9 | 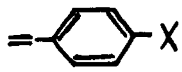 | Steroid 1b | 20,3 | 10,5 |
| 2 | Steroid 2a | 29,14 | 15 | 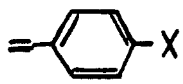 | Steroid 2b | 26,18 | 16,2 |
| 3 | Steroid 3a | 14,52 | 7,5 | 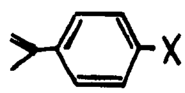 | Steroid 3b | 10,82 | 6,87 |
| 4 | Steroid 3a | 2,75 | 1,42 | 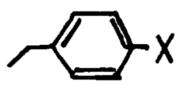 | Steroid 4a | 2,33 | 1,45 |
| 5 | Steroid 3a | 2,03 | 1,05 | 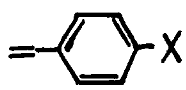 | Steroid 5a | 1,95 | 1,21 |
| 6 | Steroid 2a | 5,83 | 3,0 | 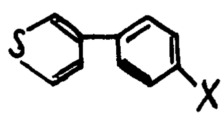 | Steroid 6b | 2,07 | 1,4 |

Tabelle 4

| Bsp. | Ansatz Stannan X | [mmol] | [g] | Halogenaromat (X=Br,I) | Ausbeute Addukt Y | [mmol] | [g] |
|---|---|---|---|---|---|---|---|
| 7 | Steroid 7a | 2.36 | 2.08 | 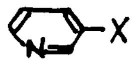 | Steroid 7b | 1.10 | 0.74 |
| 8 | Steroid 7a | 2.27 | 2.00 | 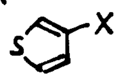 | Steroid 8b | 1.93 | 1.30 |
| 9 | Steroid 7a | 2.31 | 2.04 | 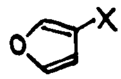 | Steroid 9b | 1.82 | 1.20 |
| 10 | Steroid 7a | 2.51 | 2.21 | 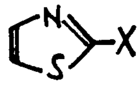 | Steroid 10b | 2.07 | 1.40 |
| 11 | Steroid 11b | 1.84 | 1.63 | 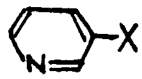 | Steroid 11c | 0.89 | 0.60 |
| 12 | Steroid 11b | 2.26 | 2.00 | 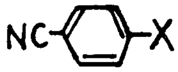 | Steroid 12b | 1.23 | 0.86 |
| 13 | Steroid 11b | 3.31 | 2.93 | 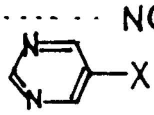 | Steroid 13b | 1.19 | 0.80 |

Tabelle 5

| Bsp. | Ansatz Halogen X | [mmol] | [g] | Heteroarylstannan (X=SnBu3) | Ausbeute Addukt Y | [mmol] | [g] |
|---|---|---|---|---|---|---|---|
| 14 | Steroid 11a | 2.22 | 1.50 | 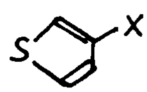 | Steroid 14b | 1.53 | 1.0 |
| 15 | Steroid 11a | 2.22 | 1.50 | 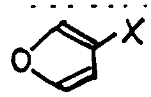 | Steroid 15b | 1.97 | 1.3 |
| 16 | Steroid 11a | 1.48 | 1.00 | 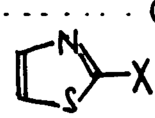 | Steroid 16b | 1.18 | 0.8 |

## Patentansprüche

1. 13-Alkyl-11$\beta$-phenyl-gonane der allgemeinen Formel I

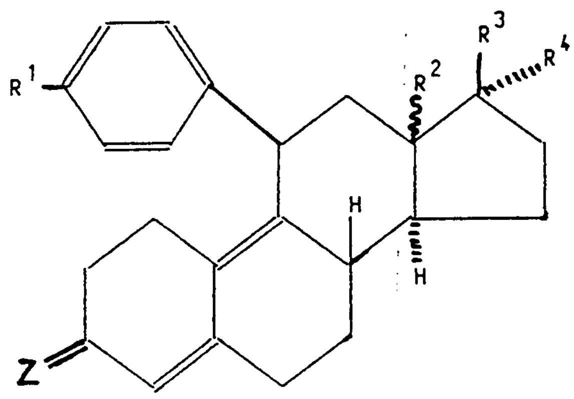

(I) ,

worin
Z für ein Sauerstoffatom oder die Hydroxyiminogruppierung N~OH,
$R^1$ entweder für
a) einen Heteroarylrest der Formel I a

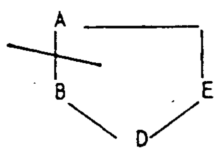

(I a),

wobei A = N, O oder S und
B-D-E die Elementenfolge
C-C-C, N-C-C oder C-N-C, ausgenommen den 1-Pyrazolrest, bedeuten oder
b) für einen Heteroarylrest der Formel I b

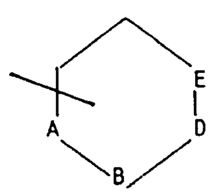

(I b) ,

wobei A = N und
B-D-E die Elementenfolge
C-C-C, N-C-C, C-N-C oder C-C-N bedeuten oder
c) für einen Cycloalkyl-, Cycloalkenyl- oder Arylrest I c oder
d) für einen geradkettigen oder verzweigten, eine oder mehrere Doppelbindungen aufweisenden Alkenylrest Id mit 2 bis 10 Kohlenstoffatomen stehen
und wobei gegebenenfalls
der Heteroarylrest der Formel I a durch einen oder mehrere Halogenreste und/oder einen oder mehrere Alkylreste mit 1 bis 3 Kohlenstoffatomen
und der Cycloalkyl-, Cycloalkenyl- oder Arylrest Ic durch eine oder mehrere Halogen-, gegebenenfalls geschützte Hydroxy-, Alkoxy-, gegebenenfalls in Form des Sulfoxids oder Sulfons und/oder des N-Oxids oxidierte Alkylthio-und/oder Dialkylaminoreste
substituiert ist/sind, und

$R^2$ für einen $\alpha$- oder $\beta$-ständigen Methyl- oder Ethylrest steht, wobei wenn $R^2$ $\alpha$-ständig ist
$R^1$ zusätzlich für einen geradkettigen oder verzweigten Alkylrest mit 1 bis 10 Kohlenstoffatomen steht und
$R^3/R^4$

$-OR_5/-C\equiv CY$

$-C\equiv CY/-OR_5$

$-OR_5/-C-CH_2R_6$
||
O

$-C-CH_2R_6/-OR_5$
||
O

$-CH_3/-C-CH_2R_6$
||
O

$-C-CH_2R_6/-CH_3$
||
O

$-OR_5/-H$

$-H/-OR_5$

$-H/-C-CH_2R_6$
||
O

$-C-CH_2R_6/H$
||
O

$-OR_5/-(CH_2)_o-CH_2R_7$

$-(CH_2)_o-CH_2R_7/-OR_5$

$-OR_5/-CH=CH-(CH_2)_kCH_2R_7$

$-CH=CH-(CH_2)_kCH_2R_7/-OR_5$

und

17 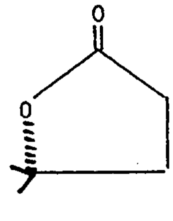 oder 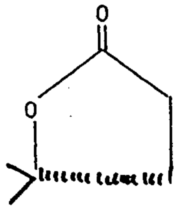

wenn $R^2$ $\beta$-ständig ist
$R^1$ zusätzlich für einen Ethylrest steht, $R^1$ aber nicht der Alkenylrest Isopropenyl sein kann und
$R^3/R^4$

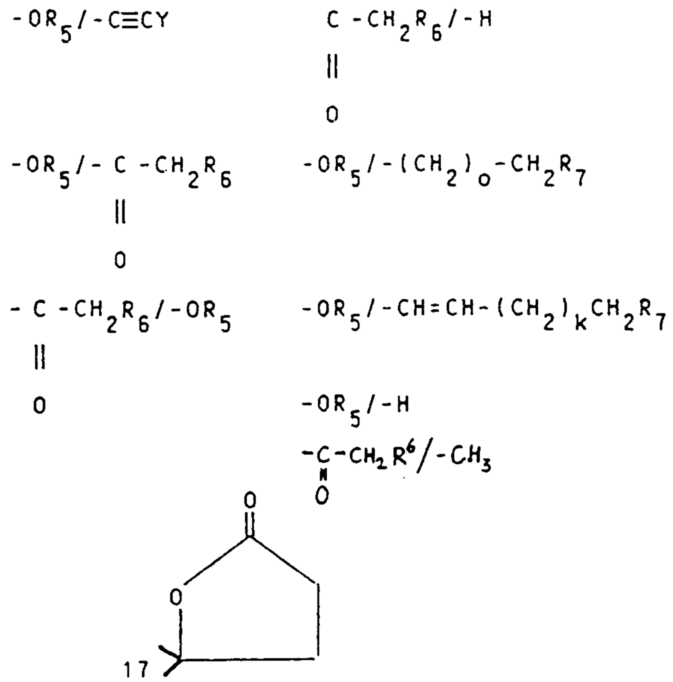

mit $R_5$ in der Bedeutung eines Wasserstoffatoms oder Acylrestes mit 1 bis 4 Kohlenstoffatomen,
Y in der Bedeutung eines Wasserstoff-, Chlor-, Fluor-, Iod- oder Bromatoms, einer Alkyl-, Hydroxyalkyl-, Alkoxyalkyl- oder Acyloxyalkylgruppe mit jeweils 1 bis 4 Kohlenstoffatomen im Alkyl- bzw. Acylrest.
$R_6$ in der Bedeutung eines Wasserstoffatoms, einer Hydroxygruppe, einer Alkyl-, O-Alkyl- oder O-Acylgruppe mit jeweils 1 bis 4 Kohlenstoffatomen.
o in der Bedeutung 0, 1, 2 oder 3,
$R_7$ in der Bedeutung eines Hydroxy- oder Cyanidrestes, einer O-Alkyl- oder O-Acylgruppe mit jeweils 1 bis 4 Kohlenstoffatomen und
k in der Bedeutung 0, 1 oder 2,
bedeuten.

**2.** 11β-(4-Ethylphenyl)-17-hydroxy-17β-(3-hydroxypropyl)-13α-methyl-4,9-gonadien-3-on
11β-(4-Ethylphenyl)-17-hydroxy-17α-(3-hydroxypropyl)-13α-methyl-4,9-gonadien-3-on
17-Hydroxy-17β-(3-hydroxypropyl)-13α-methyl-11β-(4-vinylphenyl)-4,9-gonadien-3-on
17-Hydroxy-17α-(3-hydroxypropyl)-13α-methyl-11β-(4-vinylphenyl)-4,9-gonadien-3-on
17-Hydroxy-17β-(3-hydroxypropyl)-11β-(4-isopropenylphenyl)-13α-methyl-4,9-gonadien-3-on
17-Hydroxy-17α-(3-hydroxypropyl)-11β-(4-isopropenylphenyl)-13α-methyl-4,9-gonadien-3-on
17-Hydroxy-17β-(3-hydroxypropyl)-13α-methyl-11β-[4-(3-thienyl)-phenyl]-4,9-gonadien-3-on
17-Hydroxy-17α-(3-hydroxypropyl)-13α-methyl-11β-[4-(3-thienyl)-phenyl]-4,9-gonadien-3-on
17-Hydroxy-17β-(3-hydroxypropyl)-13α-methyl-11β-[4-(2-thienyl)-phenyl]-4,9-gonadien-3-on
17-Hydroxy-17α-(3-hydroxypropyl)-13α-methyl-11β-[4-(2-thienyl)-phenyl]-4,9-gonadien-3-on
11β-[4-(2-Furyl)-phenyl]-17-hydroxy-17β-(3-hydroxypropyl)-13α-methyl-4,9-gonadien-3-on
11β-[4-(2-Furyl)-phenyl]-17-hydroxy-17α-(3-hydroxypropyl)-13α-methyl-4,9-gonadien-3-on
11β-[4-(3-Furyl)-phenyl]-17-hydroxy-17β-(3-hydroxypropyl)-13α-methyl-4,9-gonadien-3-on
11β-[4-(3-Furyl)-phenyl]-17-hydroxy-17α-(3-hydroxypropyl)-13α-methyl-4,9-gonadien-3-on
17-Hydroxy-17β-(3-hydroxypropyl)-13α-methyl-11β-[4-(2-pyridyl)-phenyl]-4,9-gonadien-3-on
17-Hydroxy-17α-(3-hydroxypropyl)-13α-methyl-11β-[4-(2-pyridyl)-phenyl]-4,9-gonadien-3-on
17-Hydroxy-17β-(3-hydroxypropyl)-13α-methyl-11β-[4-(3-pyridyl)-phenyl]-4,9-gonadien-3-on
17-Hydroxy-17α-(3-hydroxypropyl)-13α-methyl-11β-[4-(3-pyridyl)-phenyl]-4,9-gonadien-3-on
17-Hydroxy-17β-(3-hydroxypropyl)-13α-methyl-11β-[4-(4-pyridyl)-phenyl]-4,9-gonadien-3-on
17-Hydroxy-17α-(3-hydroxypropyl)-13α-methyl-11β-[4-(4-pyridyl)-phenyl]-4,9-gonadien-3-on
11β-[4-(4-Dimethylaminophenyl)-phenyl]-17-hydroxy-17β-(3-hydroxypropyl)-13α-methyl-4,9-gonadien-3-on
11β-[4-(4-Dimethylaminophenyl)-phenyl]-17-hydroxy-17α-(3-hydroxypropyl)-13α-methyl-4,9-gonadien-3-on
11β-[4-(3-Dimethylaminophenyl)-phenyl]-17-hydroxy-17β-(3-hydroxypropyl)-13α-methyl-4,9-gonadien-3-on
17-Hydroxy-17β-(3-hydroxypropyl)-13α-methyl-11β-[4-(4-cyanophenyl)-phenyl]-4,9-gonadien-3-on
11β-[4-(3-Dimethylaminophenyl)-phenyl]-17-hydroxy-17α-(3-hydroxypropyl)-13α-methyl-4,9-gonadien-3-on
11β-[4-(2-Dimethylaminophenyl)-phenyl]-17-hydroxy-17β-(3-hydroxypropyl)-13α-methyl-4,9-gonadien-3-on
11β-[4-(2-Dimethylaminophenyl)-phenyl]-17-hydroxy-17α-(3-hydroxypropyl)-13α-methyl-4,9-gonadien-3-on
17-Hydroxy-17β-(3-hydroxypropyl)-13α-methyl-11β-[4-(2-thiazolyl)-phenyl]-4,9-gonadien-3-on
17-Hydroxy-17α-(3-hydroxypropyl)-13α-methyl-11β-[4-(2-thiazolyl)-phenyl]-4,9-gonadien-3-on
11β-(4-Ethylphenyl)-17-hydroxy-17α-(3-hydroxyprop-(Z)-1-enyl)-4,9-estradien-3-on
17-Hydroxy-17α-(3-hydroxypropyl)-(Z)-1-enyl)-11β-(4-vinylphenyl)-4,9-estradien3-on
17-Hydroxy-17α-(3-hydroxypropyl)-(Z)-1-enyl)-11β-[4-(3-thienyl)-phenyl]-4,9-estradien-3-on
17-Hydroxy-17α-(3-hydroxypropyl)-(Z)-1-enyl)-11β-[4-(2-thienyl)-phenyl]-4,9-estradien-3-on
11β-(4-Ethylphenyl)-17-hydroxy-17α-(1-propinyl)-4,9-estraden-3-on
17-Hydroxy-17α-(1-propinyl)-11β-(4-vinylphenyl)-4,9-estradien-3-on.
17-Hydroxy-17α-(3-hydroxypropyl)-(Z)-1-enyl)-11β-[4-(3-furyl)-phenyl]-4,9-estradien-3-on.

**3.** 13-Alkylgonane nach Anspruch 1, worin der Heteroarylrest der Formel I a der 3-Thienyl-, 3-Furyl- oder 3-Pyrrolylrest ist.

**4.** 13-Alkylgonane nach Anspruch 1, worin der Heteroarylrest der Formel I b der 3- oder 4-Pyridyl-, der 5-Pyrimidin-, 4-Pyridazin- oder Pyrazinrest ist.

**5.** 13-Alkylgonane nach Anspruch 1, worin der Cycloalkyl-, Cycloalkenyl- oder Arylrest I c der Cyclohexyl-, ein Cyclohexenyl- oder der Phenylrest ist.

**6.** 13-Alkylgonane nach Anspruch 1, worin der Alkenylrest I d bis zu 3 Doppelbindungen aufweist.

**7.** 13-Alkylgonane nach Anspruch 1, worin der Heteroarylrest der Formel I a durch ein Chlor- Oder Bromatom substituiert ist.

**8.** 13-Alkylgonane nach Anspruch 1, worin der Heteroarylrest der Formel I a durch einen Alkylrest mit 1 bis 3 Kohlenstoffatomen substituiert ist.

**9.** 13 -Alkylgonane nach Anspruch 1, worin der Cycloalkyl-, Cycloalkenyl-oder Arylrest I c durch ein oder zwei Chlor- und/oder Bromatom(e) substituiert ist.

**10.** 13-Alkylgonane nach Anspruch 1, worin der Cycloalkyl-, Cycloalkenyl- oder Arylrest Ic durch ein oder zwei, gegebenenfalls geschützte Hydroxy- und/oder Alkoxyreste mit 1 bis 8 Kohlenstoffatomen substituiert ist.

**11.** Verfahren zur Herstellung von Verbindungen der allgemeinen Formel I

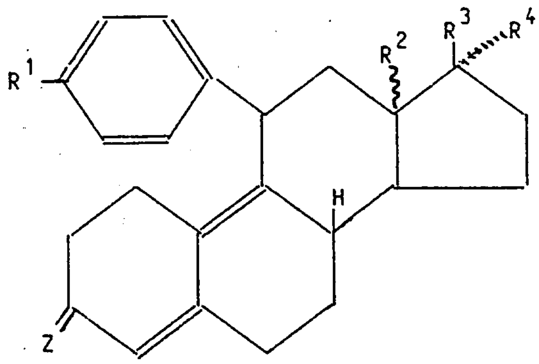

(I) ,

worin
Z für ein Sauerstoffatom oder die Hydroxyiminogruppierung N~OH,
$R^1$ entweder für

a) einen Heteroarylrest der Formel I a

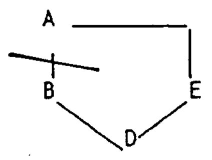

(I a),

wobei A = N, O oder S und
B-D-E die Elementenfolge
C-C-C, N-C-C oder C-N-C, ausgenommen den 1-Pyrazolrest, bedeuten oder

b) für einen Heteroarylrest der Formel I b

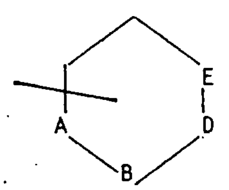

(I b) ,

wobei A = N und
B-D-E die Elementenfolge
C-C-C, N-C-C, C-N-C oder C-C-N bedeuten oder

c) für einen Cycloalkyl-, Cycloalkenyl- oder Arylrest I c oder

d) für einen geradkettigen oder verzweigten, eine oder mehrere Doppelbindungen aufweisenden Alkenylrest Id mit 2 bis 10 Kohlenstoffatomen, stehen
und wobei gegebenenfalls
der Heteroarylrest der Formel I a durch einen oder mehrere Halogenreste und/oder einen oder mehrere Alkylreste mit 1 bis 3 Kohlenstoffatomen
und der Cycloalkyl-, Cycloalkenyl- oder Arylrest Ic durch eine oder mehrere Halogen-, gegebenenfalls geschützte Hydroxy-, Alkoxy-, gegebenenfalls in Form des Sulfoxids oder Sulfons und/oder des N-Oxids oxidierte Alkylthio- und/oder Dialkylaminoreste substituiert ist/sind, und

$R^2$ für einen $\alpha$- oder $\beta$-ständigen Methyl oder Ethylrest steht, wobei wenn $R^2$ $\alpha$-ständig ist
$R^1$ zusätzlich für einen geradkettigen oder verzweigten Alkylrest mit 1 bis 10 Kohlenstoffatomen steht und
$R^3/R^4$

$-OR_5/-C{\equiv}CY$ $-H/-C-CH_2R_6$
||
O

$-C{\equiv}CY/-OR_5$ $-C-CH_2R_6/H$
||
O

$-OR_5/-C-CH_2R_6$ $-OR_5/-(CH_2)_o-CH_2R_7$
||
O

$-C-CH_2R_6/-OR_5$ $-(CH_2)_o-CH_2R_7/-OR_5$
||
O

$-CH_3/-C-CH_2R_6$ $-OR_5/-CH{=}CH-(CH_2)_kCH_2R_7$
||
O

$-C-CH_2R_6/-CH_3$ $-CH{=}CH-(CH_2)_kCH_2R_7/-OR_5$
||
O

$-OR_5/-H$
$-H/-OR_5$
und

17 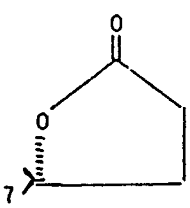 oder 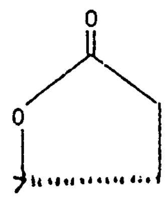

wenn $R^2$ $\beta$-ständig ist
$R^1$ zusätzlich für einen Ethylrest steht, $R^1$ aber nicht der Alkenylrest Isopropenyl sein kann und und $R^3/R^4$

$-OR_5/-C\equiv CY$

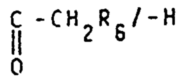

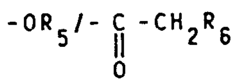

$-OR_5/-(CH_2)_o-CH_2R_7$

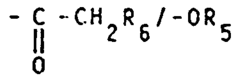

$-OR_5/-CH=CH-(CH_2)_kCH_2R_7$

$-OR_5/-H$

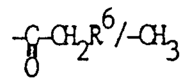

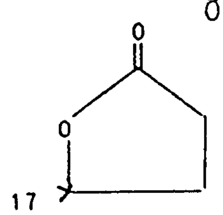

mit $R_5$ in der Bedeutung eines Wasserstoffatoms oder Acylrestes mit 1 bis 4 Kohlenstoffatomen,
Y in der Bedeutung eines Wasserstoff-, Chlor-, Fluor-, Iod- oder Bromatoms, einer Alkyl-, Hydroxylkyl-, Alkoxyalkyl- oder Acyloxyalkylgruppe mit jeweils 1 bis 4 Kohlenstoffatomen im Alkyl- bzw. Acylrest,
$R_6$ in der Bedeutung eines Wasserstoffatoms, einer Hydroxygruppe, einer Alkyl-, O-Alkyl- oder O-Acylgruppe mit jeweils 1 bis 4 Kohlenstoffatomen,
o in der Bedeutung 0, 1, 2 oder 3,
$R_7$ in der Bedeutung eines Hydroxy- oder Cyanidrestes, einer O-Alkyl- oder O-Acylgruppe mit jeweils 1 bis 4 Kohlenstoffatomen und
k in der Bedeutung 0, 1 oder 2,
dadurch gekennzeichnet. daß eine Verbindung der allgemeinen Formel II

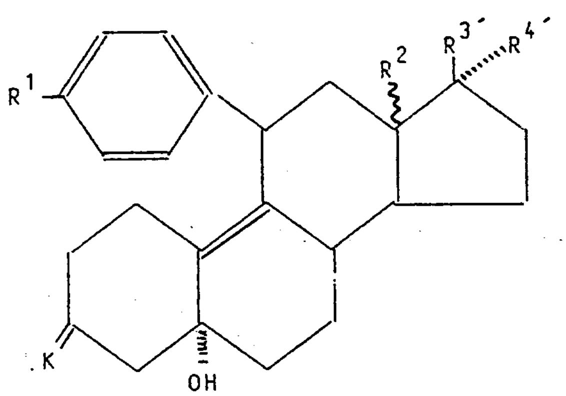

(II) ,

worin K eine in Form des Ketals, des Thioketals, des Oxims oder des Methyloxims blockierte Ketogruppe bedeutet, $R^1$ und $R^2$ die oben genannte Bedeutung haben und $R^{3'}$ und $R^{4'}$ die gleiche Bedeutung wie $R^3$ bzw. $R^4$ haben, wobei in $R^3$ gegebenenfalls vorhandene Hydroxygruppen und in $R^4$ gegebenenfalls vorhandene Hydroxy- und /oder Acylgruppen geschützt sind,
der Einwirkung eines Dehydratisierungsmittels, das auch zur Freisetzung der geschützten Funktion(en) befähigt ist, zur Wasserabspaltung unter Ausbildung der 4(5)-Doppelbindung unterworfen, im Cycloalkyl-, Cycloalkenyloder Arylrest I c gegebenenfalls vorhandene Hydroxy- und /oder Alkoxyreste gewünschtenfalls erneut geschützt, im. Rest I c gegebenenfalls vorhandene Alkylthio- und /oder Dialkylaminoreste gewünschtenfalls zum entsprechenden Sulfoxid, Sulfon und /oder N-Oxid oxidiert und gewünschtenfalls anschließend mit Hydroxylamin-hydrochlorid in Gegenwart von tertiären Aminen bei Temperaturen zwischen -20 °C und +40 °C umgesetzt wird.

**12.** Verfahren zur Herstellung der Ausgangsprodukte der allgemeinen Formel II

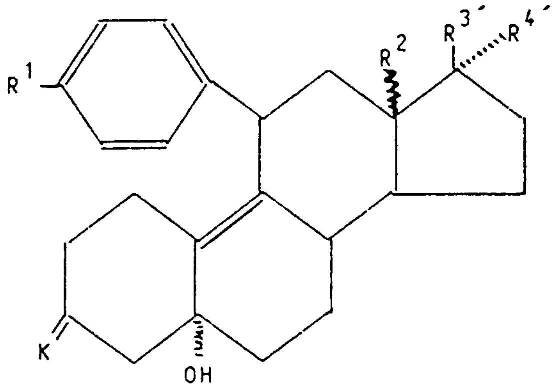

(II) ,

worin K, $R^1$, $R^2$, $R^{3'}$ und $R^{4'}$ die angegebene Bedeutung haben, dadurch gekennzeichnet, daß eine Verbindung der allgemeinen Formel III

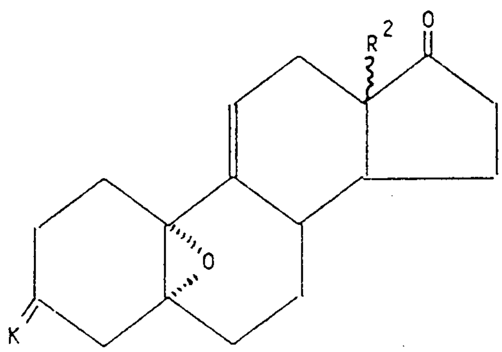

(III) ,

worin K und $R^2$ die angegebene Bedeutung haben, nach an sich bekannten Verfahren des C-17-Seitenkettenaufbaues in eine Verbindung der allgemeinen Formel IV

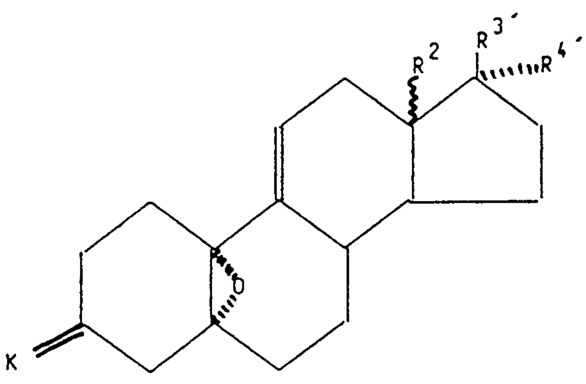

(IV) ,

worin K, $R^2$, $R^{3'}$ und $R^{4'}$ die bereits angegebene Bedeutung haben, überführt wird, und diese Verbindung der allgemeinen Formel IV anschließend entweder durch Grignard-Reaktion mit einem entsprechenden Arylmagnesiumhalogenid

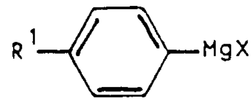

(X = Br, I) in eine Verbindung der allgemeinen Formel II überführt oder durch Alkylierung mit einer Verbindung des Typs

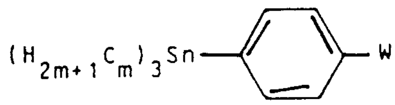

worin W = MgX (X = Br, I) oder vorzugsweise Li und m = 1, 2, 3, 4, vorzugsweise 1 oder 4 bedeuten,
in eine Verbindung der allgemeinen Formel V

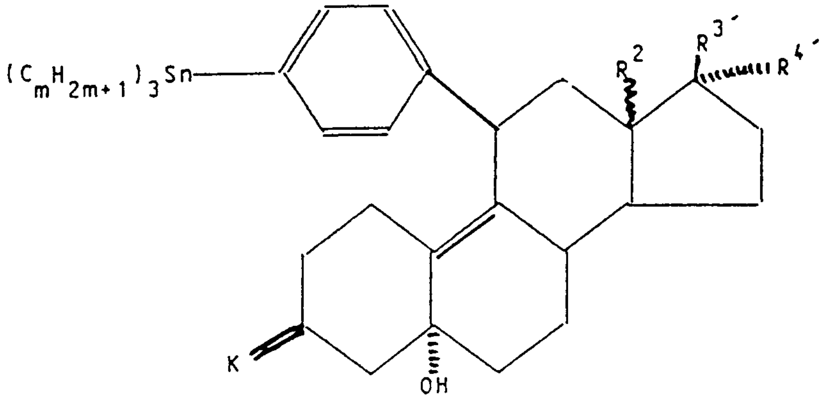

(V) ,

worin alle Substituenten die bereits angegebene Bedeutung haben, umgewandelt,
diese anschließend in einer übergangsmetall-, vorzugsweise Pd(O)-katalysierten Reaktion in Gegenwart einer Verbindung $R^1$-Y, worin $R^1$ den im Ausgangsprodukt der allgemeinen Formel II gewünschten Substituenten $R^1$ und Y Halogen, vorzugsweise Brom, bedeuten, zu einer Verbindung der allgemeinen Formel II umgesetzt oder
wenn $R^2$-$\alpha$-ständig durch Grignard-Reaktion mit einem entsprechenden Halogenarylmagnesiumhalogenid

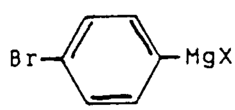

(X = Br, I) in eine Verbindung der allgemeinen Formel VI

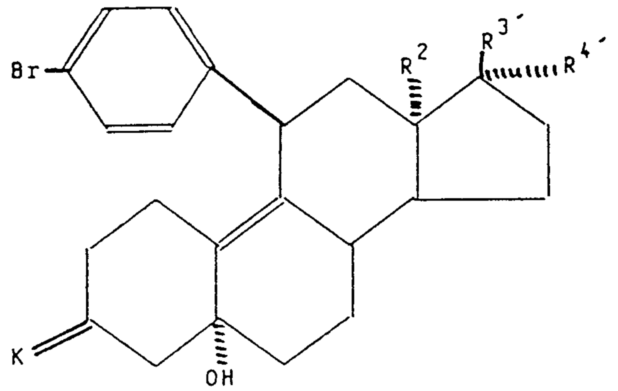

(VI) ,

worin K, $R^2$, $R^{3'}$ und $R^{4'}$ die oben angegebene Bedeutung haben, umgewandelt und dann die Verbindung der allgemeinen Formel VI zur Einführung des $R^1$ -Substituenten in die 4-Position des 11β-konfigurierten Phenylringes übergangsmetallkatalysiert, vorzugsweise Pd(O)-katalysiert mit einer zinnorganischen Verbindung der allgemeinen Formel $R^1Sn(C_mH_{2m+1})_3$ reagieren gelassen wird, wobei eine Verbindung der allgemeinen Formel II entsteht oder aber eine Verbindung der allgemeinen Formel VI zunächst übergangsmetallkatalysiert, vorzugsweise Pd(O)-katalysiert, mit einem Hexaalkyldistannan [-$(C_mH_{2m+1})_3Sn]_2$ (m = 1, 2, 3, 4; vorzugsweise 1 oder 4) zu einer Verbindung der allgemeinen Formel V und diese anschließend - wie bereits angegeben - zu einer Verbindung der allgemeinen Formel II weiterverarbeitet wird.

**13.** Verfahren zur Herstellung von Verbindungen der allgemeinen Formel II

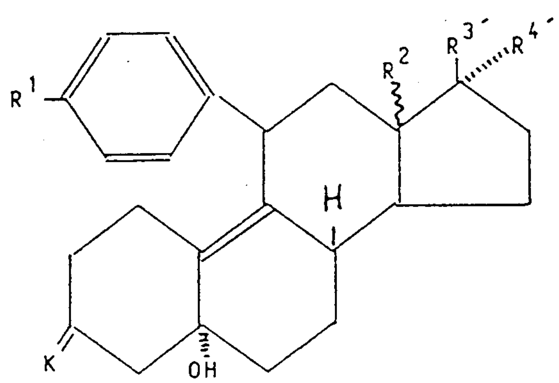

(II) ,

worin K, $R^1$, $R^2$, $R^{3'}$ und $R^{4'}$ die bereits angegebene Bedeutung haben, dadurch gekennzeichnet, daß zunächst eine Verbindung der allgemeinen Formel VII

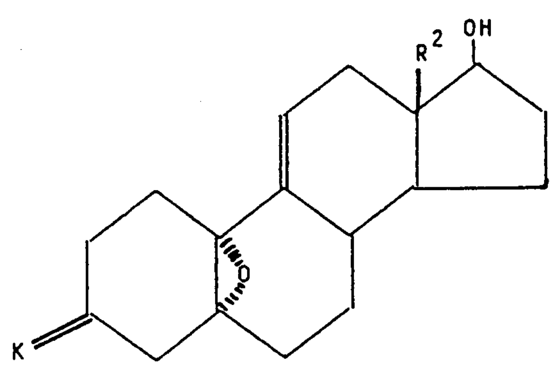

(VII) ,

worin K und $R^2$ die bereits angegebene Bedeutung haben, mit einer Verbindung der Formel

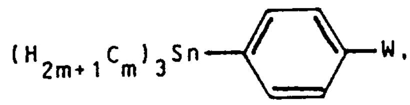

worin W = MgX (X = Br, I) oder vorzugsweise Li und
m = 1, 2, 3, 4, vorzugsweise 1 oder 4 bedeuten,
in 11-position unter Bildung einer Verbindung der allgemeinen Formel VIII

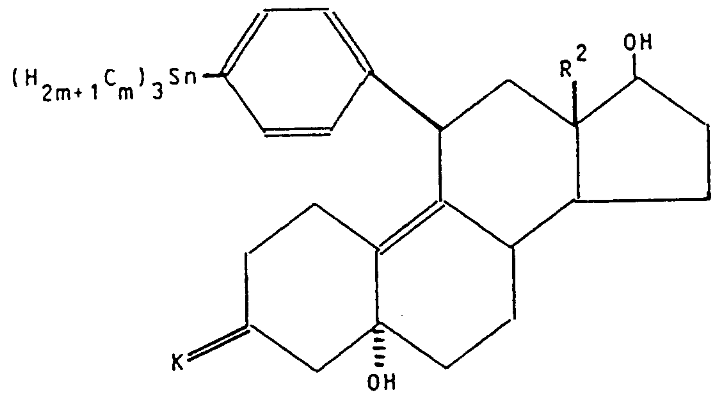

(VIII).

alkyliert wird,
diese durch übergangsmetallkatalysierte, vorzugsweise Pd(o)-katalysierte Kupplung mit einer Verbindung $R^1$-Y (Y = Br, I) zu einer Verbindung der allgemeinen Formel IX

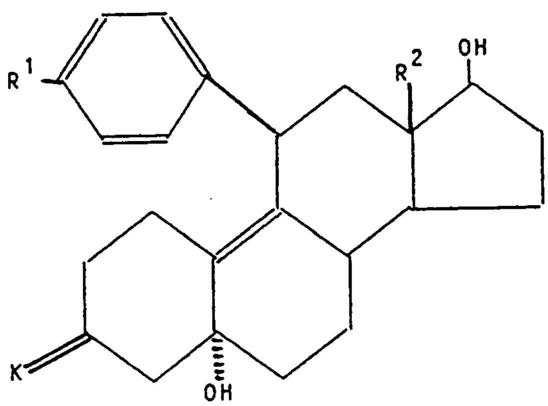

(IX) ,

umgesetzt und anschließend durch Oxidation der 17-$\beta$-OH-Funktion und gegebenenfalls nachfolgender Bestrahlung mit ultraviolettem Licht in eine Verbindung der allgemeinen Formel X

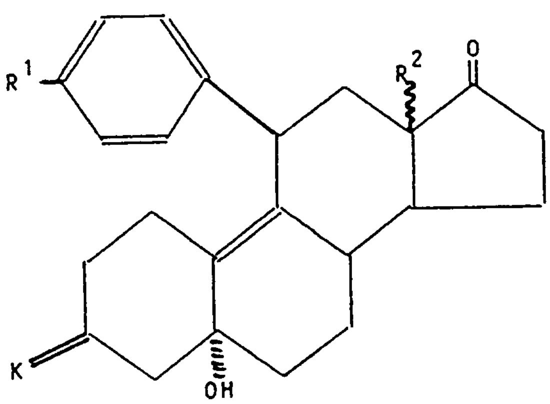

(X) ,

worin die Substituenten die bereits angegebene Bedeutung haben und $R^2$ sowohl $\alpha$- als auch $\beta$-ständig sein kann, überführt und dann in X der $R^3$-und $R^4$- bzw. $R^{3'}$ und/oder $R^{4'}$ -Substituent durch nucleophile Addition (Seitenkettenaufbau) unter Bildung einer Verbindung der allgemeinen Formel II

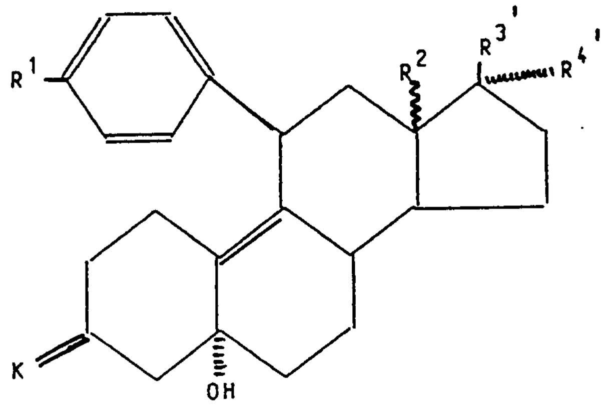

(II) ,

eingebaut wird
oder eine Verbindung der allgemeinen Formel VIII in 17-Position zuerst zu einer Verbindung der allgemeinen Formel XI

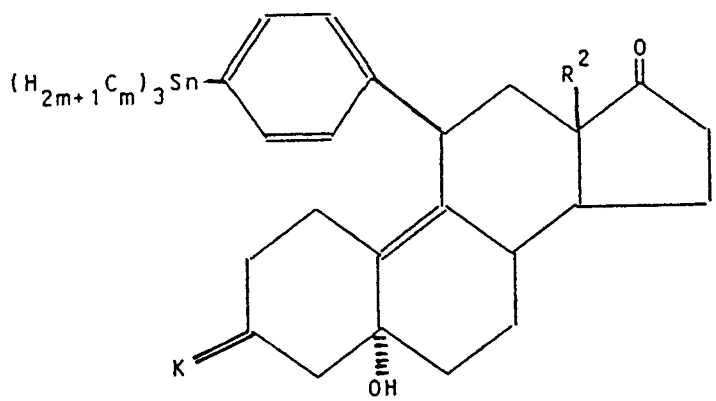

(XI)

oxidiert
und dann diese durch nucleophile Addition (Seitenkettenaufbau), gegebenenfalls nach ultravioletter Bestrahlung, über eine Verbindung der allgemeinen Formel V und durch übergangsmetallkatalysierte, vorzugsweise Pd-(O)-katalysierte Kopplung mit $R^1$-Y (Y = Br, I) zu einer Verbindung der allgemeinen Formel II weiterverarbeitet wird.

**14.** Pharmazeutische Präparate, gekennzeichnet durch einen Gehalt an Verbindungen gemäß den Ansprüchen 1 bis 10.

**15.** Verwendung von Verbindungen gemaß den Ansprüchen 1 bis 10 zur Herstellung von Arzneimitteln.

**Claims**

1. 13-Alkyl-11β-phenyl-gonanes of the general formula I

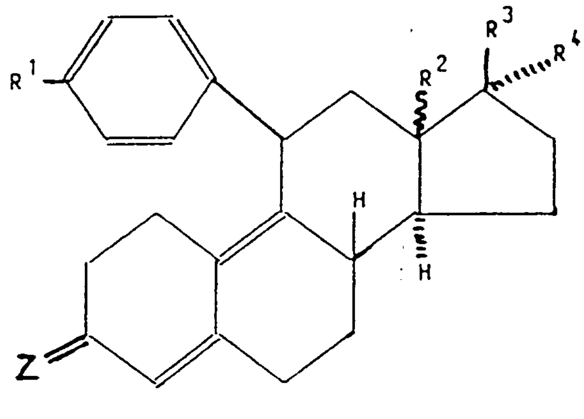

(I),

wherein
Z is an oxygen atom or the hydroxyimino group N~OH,
$R^1$ is
a) a heteroaryl radical of formula Ia

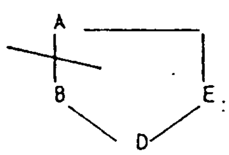

(Ia),

wherein A = N, O or S, and
B-D-E represents the sequence of elements C-C-C, N-C-C or C-N-C, with the exception of the 1-pyrazole radical, or
b) is a heteroaryl radical of formula Ib

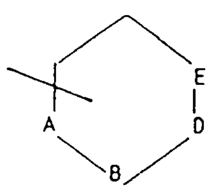

(Ib),

wherein A = N and
B-D-E represents the sequence of elements C-C-C, N-C-C, C-N-C or C-C-N, or
c) is a cycloalkyl, cycloalkenyl or aryl radical Ic, or
d) is a straight-chained or branched alkenyl radical Id having from 2 to 10 carbon atoms and one or more double bonds,
and wherein optionally
the heteroaryl radical of formula Ia is substituted by one or more halogen atoms and/or by one or more alkyl radicals having from 1 to 3 carbon atoms, and
the cycloalkyl, cycloalkenyl or aryl radical Ic is substituted by one or more halogen atoms, optionally protected hydroxy radicals, alkoxy radicals, alkylthio and/or dialkylamino radicals optionally oxidised in the form of the sulphoxide or sulphone and/or of the N-oxide, and
$R^2$ is a methyl or ethyl radical in the α- or β-configuration,
wherein, when $R^2$ is in the α-configuration,
$R^1$ additionally represents a straight-chained or branched alkyl radical having from 1 to 10 carbon atoms, and

$R^3/R^4$ is

$-OR_5/-C\equiv CY$

$-C\equiv CY/-OR_5$

$-H/-C-CH_2R_6$
||
O

$-C-CH_2R_6/H$
||
O

$-OR_5/-C-CH_2R_6$
||
O

$-C-CH_2R_6/-OR_5$
||
O

$-OR_5/-(CH_2)_o-CH_2R_7$

$-(CH_2)_o-CH_2R_7/-OR_5$

$-CH_3/-C-CH_2R_6$
||
O

$-C-CH_2R_6/-CH_3$
||
O

$-OR_5/-CH=CH-(CH_2)_kCH_2R_7$

$-CH=CH-(CH_2)_kCH_2R_7/-OR_5$

$-OR_5/-H$

$-H/-OR_5$

and

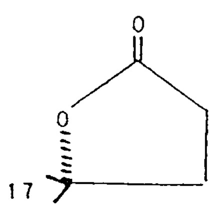

or

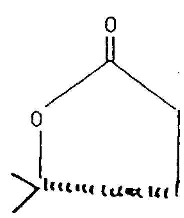

and, when $R^2$ is in the $\beta$-configuration,

$R^1$ additionally represents an ethyl radical, but $R^1$ cannot be the alkenyl radical isopropenyl, and $R^3/R^4$ is

$-OR_5/-C\equiv CY$

$\underset{\underset{O}{\|}}{C}-CH_2R_6/-H$

$-OR_5/-\underset{\underset{O}{\|}}{C}-CH_2R_6$

$-OR_5/-(CH_2)_o-CH_2R_7$

$-\underset{\underset{O}{\|}}{C}-CH_2R_6/-OR_5$

$-OR_5/-CH=CH-(CH_2)_kCH_2R_7$

$-OR_5/-H$

$-\underset{\underset{O}{\|}}{C}-CH_2R^6/-CH_3$

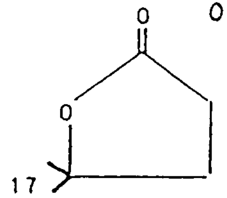

wherein $R_5$ is a hydrogen atom or an acyl radical having from 1 to 4 carbon atoms,
Y is a hydrogen, chlorine, fluorine, iodine or bromine atom, or an alkyl, hydroxyalkyl, alkoxyalkyl or acyloxyalkyl group each having from 1 to 4 carbon atoms in the alkyl or acyl radical,
$R_6$ is a hydrogen atom, a hydroxy group, or an alkyl, O-alkyl or O-acyl group each having from 1 to 4 carbon atoms,
$\underline{o}$ is 0, 1, 2 or 3,
$R_7$ is a hydroxy or cyanide radical, or an O-alkyl or O-acyl group each having from 1 to 4 carbon atoms, and
$\underline{k}$ is 0, 1 or 2.

**2.** 11β-(4-ethylphenyl)-17-hydroxy-17β-(3-hydroxypropyl)-13α-methyl-4,9-gonadien3-one
11β-(4-ethylphenyl)-17-hydroxy-17α-(3-hydroxypropyl)-13α-methyl-4,9-gonadien-3-one
17-hydroxy-17β-(3-hydroxypropyl)-13α-methyl-11β-(4-vinylphenyl)-4,9-gonadien-3-one
17-hydroxy-17α-(3-hydroxypropyl)-13α-methyl-11β-(4-vinylphenyl)-4,9-gonadien-3-one
17-hydroxy-17β-(3-hydroxypropyl)-11β-(4-isopropenylphenyl)-13α-methyl-4,9-gonadien-3-one
17-hydroxy-17α-(3-hydroxypropyl)-11β-(4-isopropenylphenyl)-13α-methyl-4,9-gonadien-3-one
17-hydroxy-17β-(3-hydroxypropyl)-13α-methyl-11β-[4-(3-thienyl)phenyl]-4,9-gonadien-3-one
17-hydroxy-17α-(3-hydroxypropyl)-13α-methyl-11β-[4-(3-thienyl)phenyl]-4,9-gonadien-3-one
17-hydroxy-17β-(3-hydroxypropyl)-13α-methyl-11β-[4-(2-thienyl)phenyl]-4,9-gonadien-3-one
17-hydroxy-17α-(3-hydroxypropyl)-13α-methyl-11β-[4-(2-thienyl)phenyl]-4,9-gonadien-3-one
11β-[4-(2-furyl)phenyl]-17-hydroxy-17β-(3-hydroxypropyl)-13α-methyl-4,9-gonadien-3-one
11β-[4-(2-furyl)phenyl]-17-hydroxy-17α-(3-hydroxypropyl)-13α-methyl-4,9-gonadien-3-one
11β-[4-(3-furyl)phenyl]-17-hydroxy-17β-(3-hydroxypropyl)-13α-methyl-4,9-gonadien-3-one
11β-[4-(3-furyl)phenyl]-17-hydroxy-17α-(3-hydroxypropyl)-13α-methyl-4,9-gonadien-3-one
17-hydroxy-17β-(3-hydroxypropyl)-13α-methyl-11β-[4-(2-pyridyl)phenyl]-4,9-gonadien-3-one
17-hydroxy-17α-(3-hydroxypropyl)-13α-methyl-11β-[4-(2-pyridyl)phenyl]-4,9-gonadien-3-one
17-hydroxy-17β-(3-hydroxypropyl)-13α-methyl-11β-[4-(3-pyridyl)phenyl]-4,9-gonadien-3-one
17-hydroxy-17α-(3-hydroxypropyl)-13α-methyl-11β-[4-(3-pyridyl)phenyl]-4,9-gonadien-3-one
17-hydroxy-17β-(3-hydroxypropyl)-13α-methyl-11β-[4-(4-pyridyl)phenyl]-4,9-gonadien-3-one
17-hydroxy-17α-(3-hydroxypropyl)-13α-methyl-11β-[4-(4-pyridyl)phenyl]-4,9-gonadien-3-one
11β-[4-(4-dimethylaminophenyl)phenyl]-17-hydroxy-17β-(3-hydroxypropyl)-13α-methyl-4,9-gonadien-3-one
11β-[4-(4-dimethylaminophenyl)phenyl]-17-hydroxy-17α-(3-hydroxypropyl)-13α-methyl-4,9-gonadien-3-one

11β-[4-(4-dimethylaminophenyl)phenyl]-17-hydroxy-17β-(3-hydroxypropyl)-13α-methyl-4,9-gonadien-3-one
17-hydroxy-17β-(3-hydroxypropyl)-13α-methyl-11β-[4-(4-cyanophenyl)phenyl]-4,9-gonadien-3-one
11β-[4-(3-dimethylaminophenyl)phenyl]-17-hydroxy-17α-(3-hydroxypropyl)-13α-methyl-4,9-gonadien-3-one
11β-[4-(2-dimethylaminophenyl)phenyl]-17-hydroxy-17β-(3-hydroxypropyl)-13α-methyl-4,9-gonadien-3-one
11β-[4-(2-dimethylaminophenyl)phenyl]-17-hydroxy-17α-(3-hydroxypropyl)-13α-methyl-4,9-gonadien-3-one
17-hydroxy-17β-(3-hydroxypropyl)-13α-methyl-11β-[4-(2-thiazolyl)phenyl]-4,9-gonadien-3-one
17-hydroxy-17α-(3-hydroxypropyl)-13α-methyl-11β-[4-(2-thiazolyl)phenyl]-4,9-gonadien-3-one
11β-(4-ethylphenyl)-17-hydroxy-17α-(3-hydroxyprop-(Z)-1-enyl)-4,9-oestradien-3-one
17-hydroxy-17α-(3-hydroxypropyl)-(Z)-1-enyl)-11β-(4-vinylphenyl)-4,9-oestradien-3-one
17-hydroxy-17α-(3-hydroxypropyl)-(Z)-1-enyl)-11β-[4-(3-thienyl)phenyl)-4,9-oestradien-3-one
17-hydroxy-17α-(3-hydroxypropyl)-(Z)-1-enyl)-11β-[4-(2-thienyl)phenyl)-4,9-oestradien-3-one
11β-(4-ethylphenyl)-17-hydroxy-17α-(1-propynyl)-4,9-oestradien-3-one
17-hydroxy-17α-(1-propynyl)-11β-(4-vinylphenyl)-4,9-oestradien-3-one
17-hydroxy-17α-(3-hydroxyprop-(Z)-1-enyl)-11β-[4-(3-furyl)phenyl]-4,9-oestradien-3-one.

**3.** 13-Alkyl-gonanes according to claim 1, wherein the heteroaryl radical of formula Ia is the 3-thienyl, 3-furyl or 3-pyrrolyl radical.

**4.** 13-Alkyl-gonanes according to claim 1, wherein the heteroaryl radical of formula Ib is the 3- or 4-pyridyl, 5-pyrimidine, 4-pyridazine or pyrazine radical.

**5.** 13-Alkyl-gonanes according to claim 1, wherein the cycloalkyl, cycloalkenyl or aryl radical Ic is the cyclohexyl radical, a cyclohexenyl radical or the phenyl radical.

**6.** 13-Alkyl-gonanes according to claim 1, wherein the alkenyl radical Id has a maximum of 3 double bonds.

**7.** 13-Alkyl-gonanes according to claim 1, wherein the heteroaryl radical of formula Ia is substituted by a chlorine or bromine atom.

**8.** 13-Alkyl-gonanes according to claim 1, wherein the heteroaryl radical of formula Ia is substituted by an alkyl radical having from 1 to 3 carbon atoms.

**9.** 13-Alkyl-gonanes according to claim 1, wherein the cycloalkyl, cycloalkenyl or aryl radical Ic is substituted by one or two chlorine and/or bromine atoms.

**10.** 13-Alkyl-gonanes according to claim 1, wherein the cycloalkyl, cycloalkenyl or aryl radical Ic is substituted by one or two optionally protected hydroxy radicals and/or alkoxy radicals having from 1 to 8 carbon atoms.

**11.** Process for the preparation of compounds of the general formula I

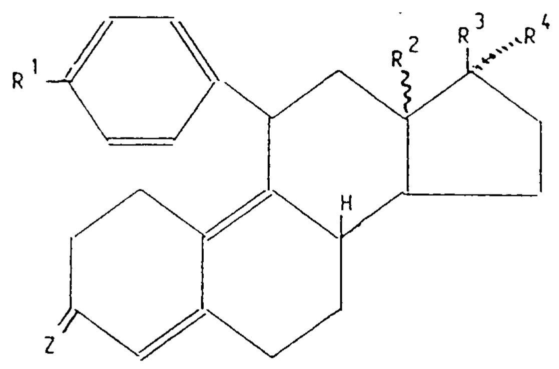

(I),

wherein
Z is an oxygen atom or the hydroxyimino group N~OH,
$R^1$ is
a) a heteroaryl radical of formula Ia

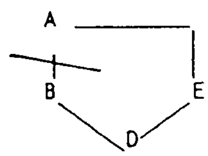

(Ia),

wherein
A = N, O or S, and
B-D-E represents the sequence of elements C-C-C, N-C-C or C-N-C, with the exception of the 1-pyrazole radical, or
b) is a heteroaryl radical of formula Ib

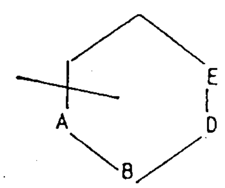

(Ib),

wherein
A = N, and
B-D-E represents the sequence of elements C-C-C, N-C-C, C-N-C or C-C-N, or
c) is a cycloalkyl, cycloalkenyl or aryl radical Ic, or
d) is a straight-chained or branched alkenyl radical Id having from 2 to 10 carbon atoms and one or more double bonds,
and wherein optionally
the heteroaryl radical of formula Ia is substituted by one or more halogen atoms and/or by one or more alkyl radicals having from 1 to 3 carbon atoms and the cycloalkyl, cycloalkenyl or aryl radical Ic is substituted by one or more halogen atoms, optionally protected hydroxy radicals, alkoxy radicals, alkylthio and/or dialkylamino radicals optionally oxidised in the form of the sulphoxide or sulphone and/or of the N-oxide, and
$R^2$ is a methyl or ethyl radical in the $\alpha$- or $\beta$-configuration,
wherein, when $R^2$ is in the $\alpha$-configuration,
$R^1$ additionally represents a straight-chained or branched alkyl radical having from 1 to 10 carbon atoms, and

$R^3/R^4$ is

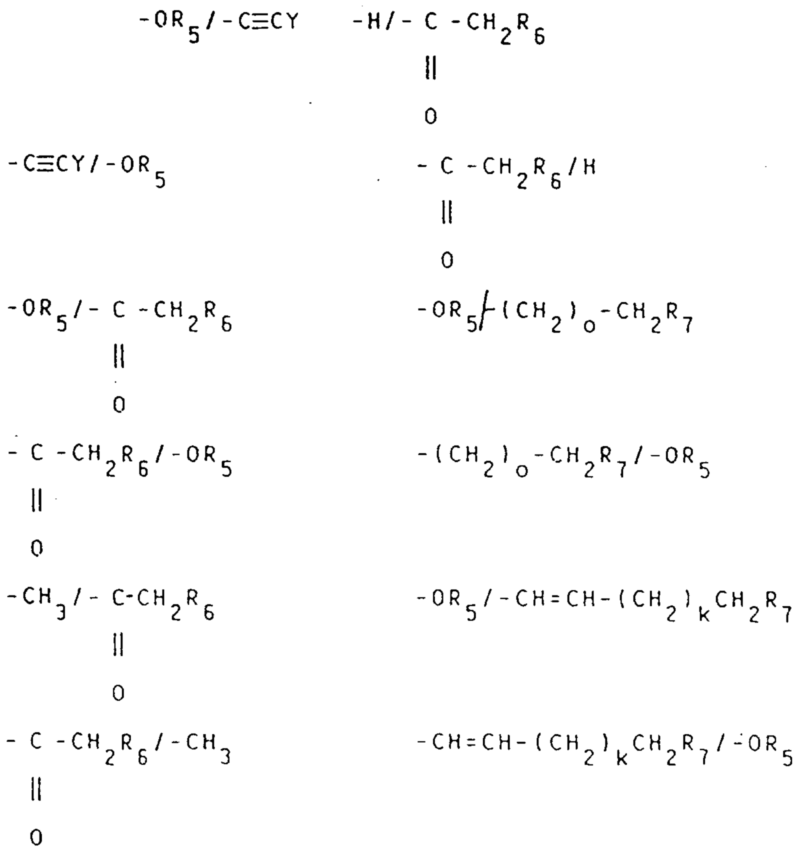

$-OR_5/-H$

$-H/-OR_5$

and

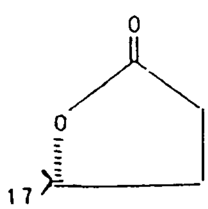 or 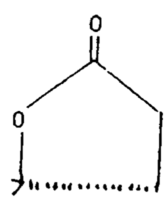

and, when $R^2$ is in the $\beta$-configuration,
$R^1$ additionally represents an ethyl radical, but $R^1$ cannot be the alkenyl radical isopropenyl, and $R^3/R^4$ is

$-OR_5/-C\equiv CY$

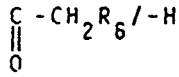

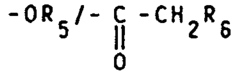

$-OR_5/-(CH_2)_o-CH_2R_7$

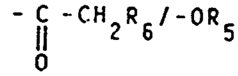

$-OR_5/-CH=CH-(CH_2)_kCH_2R_7$

$-OR_5/-H$

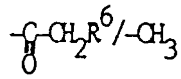

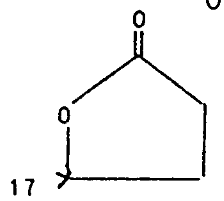

wherein $R_5$ is a hydrogen atom or an acyl radical having from 1 to 4 carbon atoms,
Y is a hydrogen, chlorine, fluorine iodine or bromine atom, or an alkyl, hydroxyalkyl, alkoxyalkyl or acyloxyalkyl group each having from 1 to 4 carbon atom in the alkyl or acyl radical,
$R_6$ is a hydrogen atom, a hydroxy group or an alkyl, O-alkyl or O-acyl group each having from 1 to 4 carbon atoms,
<u>o</u> is 0, 1, 2 or 3,
$R_7$ is a hydroxy or cyanide radical, or an O-alkyl or O-acyl group each having from 1 to 4 carbon atoms, and
<u>k</u> is 0, 1 or 2,
which process is characterized in that a compound of the general formula II

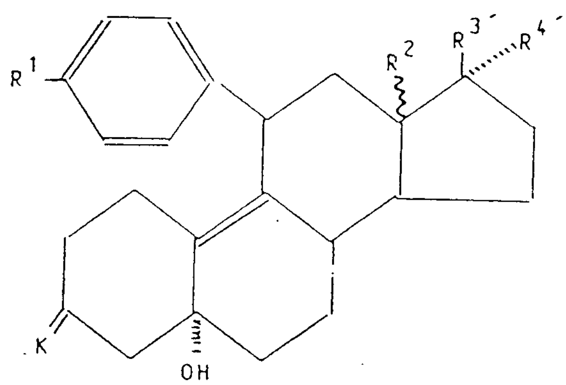

(II),

wherein
K is a blocked keto group in the form of the ketal, the thioketal, the oxime or the methyloxime,
$R^1$ and $R^2$ have the meanings given above, and
$R^{3'}$ and $R^{4'}$ have the same meanings as $R^3$ and $R^4$ respectively, any hydroxy groups present in $R^3$ and any hydroxy and/or acyl groups present in $R^4$ being protected, is subjected to the action of a dehydrating agent that is also capable of releasing the protected function(s), to remove water with the formation of the 4(5)-double bond, any hydroxy and/or alkoxy radicals present in the cycloalkyl, cycloalkenyl or aryl radical Ic are, if desired, protected again, any alkylthio and/or dialkylamino radicals present in the radical Ic are, if desired, oxidised to the corresponding sulphoxide, sulphone and/or N-oxide, and, if desired, the product is then reacted with hydroxylamine hydrochloride in the presence of tertiary amines at a temperature of from -20 °C to +40 °C.

**12.** Process for the preparation of the starting materials of the general formula II

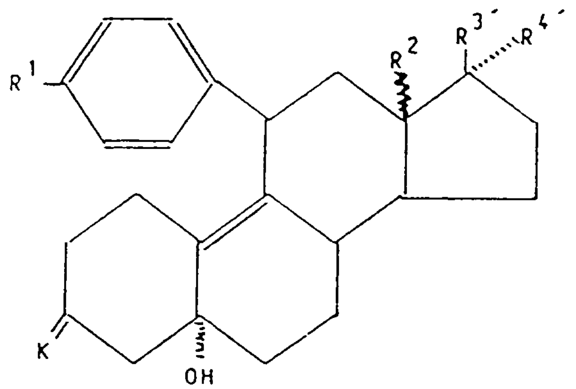

(II)

wherein
K, $R^1$, $R^2$, $R^{3'}$ and $R^{4'}$ have the meanings given above,
characterised in that a compound of the general formula III

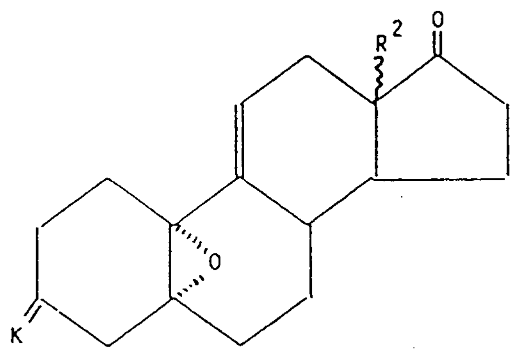

(III)

wherein K and $R^2$ have the meanings given, is converted in accordance with C-17 side chain synthesis processes known per se into a compound of the general formula IV

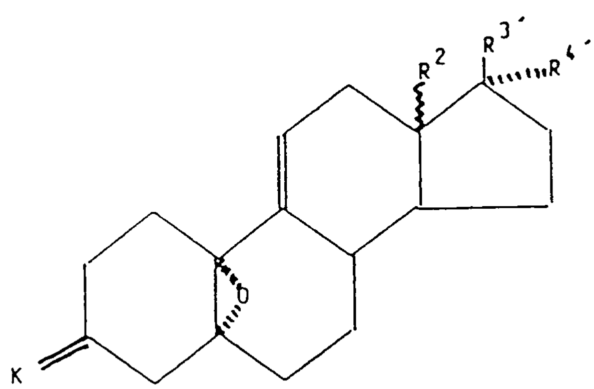

(IV)

wherein K, $R^2$, $R^{3'}$ and $R^{4'}$ have the meanings already given, and that compound of the general formula IV is then either converted by Grignard reaction with a corresponding arylmagnesium halide

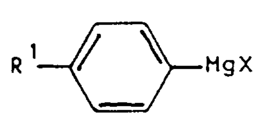

(X = Br, I) into a compound of the general formula II, or is converted by alkylation with a compound of the type

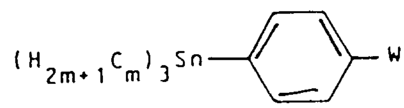

wherein W = MgX (X = Br, I) or preferably Li, and m = 1, 2, 3 or 4, preferably 1 or 4, into a compound of the general formula V

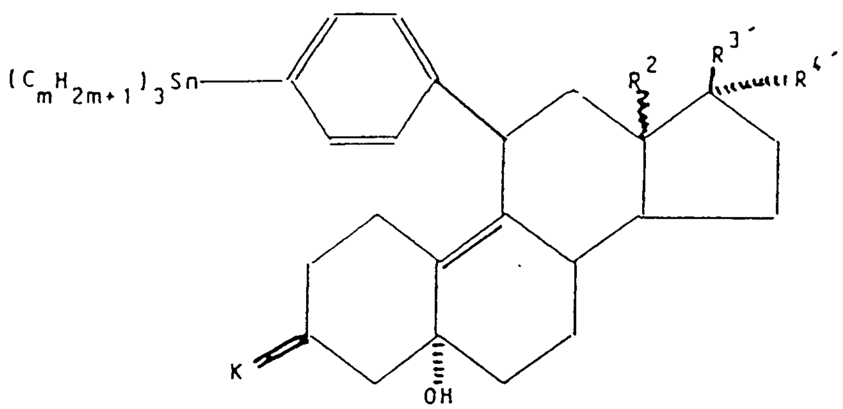

(V)

wherein all the substituents have the meanings already given,
that compound is then converted in a transition metal-catalysed, preferably Pd(O)-catalysed, reaction in the presence of a compound $R^1$-Y wherein $R^1$ represents the desired substituent $R^1$ in the starting material of the general formula II and Y is halogen, preferably bromine, into a compound of the general formula II, or, when $R^2$ is in the α-configuration, is converted by Grignard reaction with a corresponding haloarylmagnesium halide

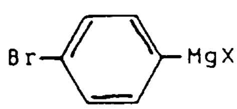

(X = Br, I) into a compound of the general formula VI

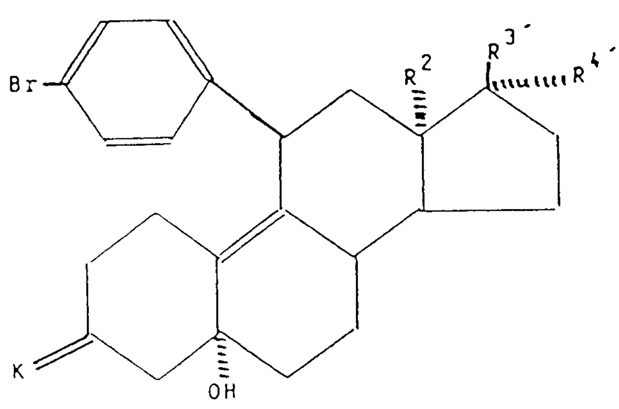

(VI)

wherein K, $R^2$, $R^{3'}$ and $R^{4'}$ have the meanings given above, and then, for the introduction of the $R^1$

substituent into the 4-position of the 11β-configured phenyl ring, the compound of the general formula VI is reacted, catalysed by a transition metal, preferably by Pd(O), with an organo-tin compound of the general formula $R^1Sn(C_mH_{2m+1})_3$ resulting in a compound of the general formula II, or, a compound of the general formula VI is first of all reacted, catalysed by a transition metal, preferably by Pd(O), with a hexaalkyldistannane $[(C_mH_{2m+1})_3Sn]_2$ (m = 1, 2, 3 or 4; preferably 1 or 4) to form a compound of the general formula V and that compound is then - as already specified - processed further to form a compound of the general formula II.

**13.** Process for the preparation of compounds of the general formula II

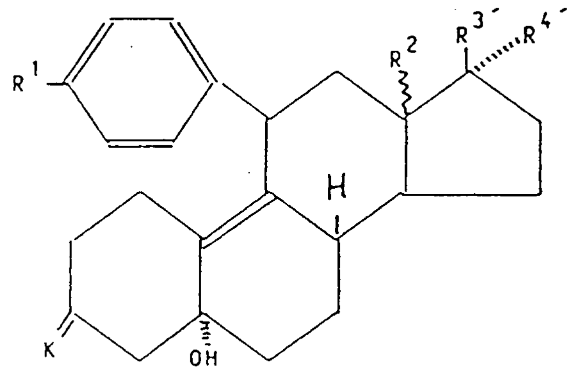

(II)

wherein K, $R^1$, $R^2$, $R^{3'}$ and $R^{4'}$ have the meanings already given, characterised in that first of all a compound of the general formula VII

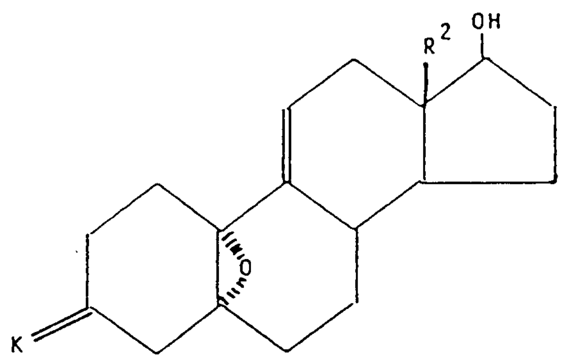

(VII)

wherein K and $R^2$ have the meanings already given, is alkylated with a compound of formula

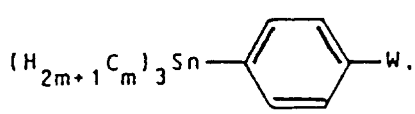

wherein W = MgX (X = Br, I) or preferably Li, and
m = 1, 2, 3 or 4, preferably 1 or 4,
in the 11-position to form a compound of the general formula VIII

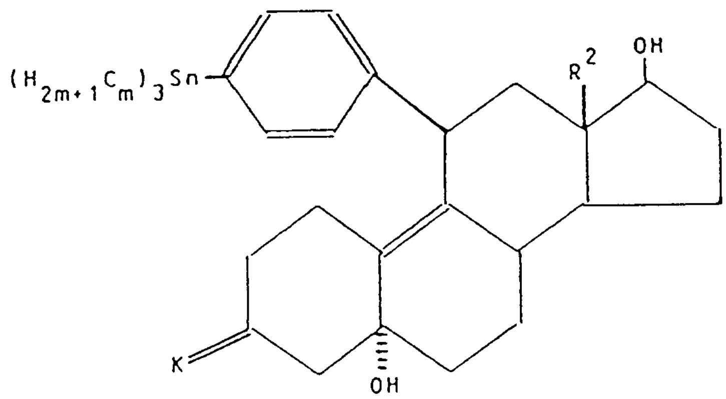

(VIII),

the latter is reacted, by transition metal-catalysed, preferably Pd(O)-catalysed, coupling, with a compound $R^1$-Y (Y = Br, I) to form a compound of the general formula IX

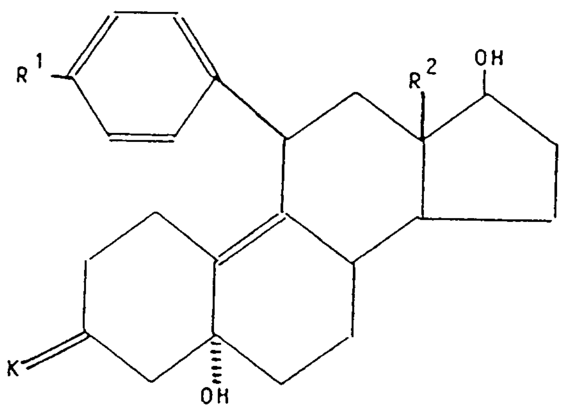

(IX)

and is then converted by oxidation of the 17$\beta$-OH function and optionally subsequent irradiation with ultraviolet light into a compound of the general formula X

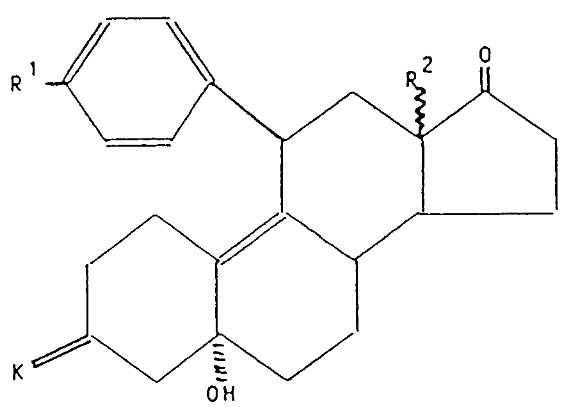

(X)

wherein the substituents have the meanings already given and $R^2$ may be in the $\alpha$- or $\beta$-configuration, and then the $R^3$ and $R^4$ or $R^{3'}$ and/or $R^{4'}$ substituent is incorporated into X by nucleophilic addition (side chain synthesis) to form a compound of the general formula II

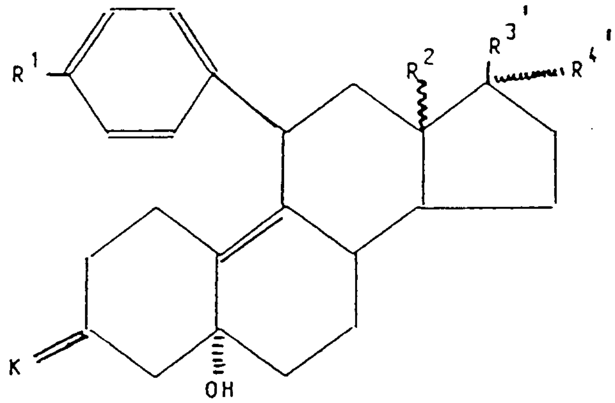

(II),

or, a compound of the general formula VIII is first oxidised in the 17-position to a compound of the general formula XI

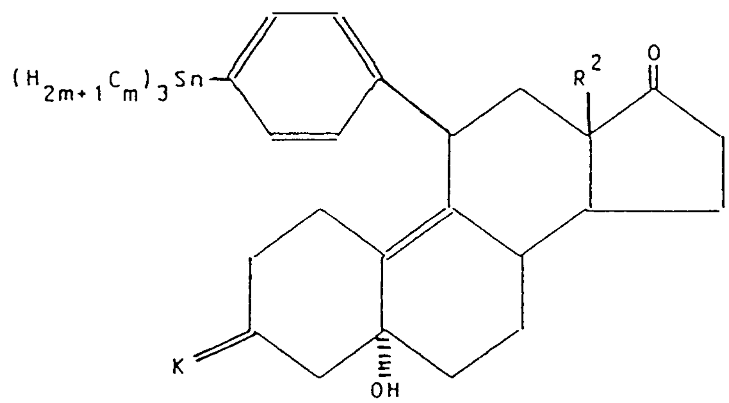

(XI)

and then the latter is processed further by nucleophilic addition (side chain synthesis), optionally after ultraviolet irradiation, by way of a compound of the general formula V and by transition metal-catalysed, preferably Pd(O)-catalysed, coupling with $R^1$-Y (Y = Br, I) to form a compound of the general formula II.

**14.** Pharmaceutical preparations, characterised in that they comprise compounds according to claims 1 to 10.

**15.** Use of compounds according to claims 1 to 10 for the preparation of medicaments.

## Revendications

**1.** 13-alkyl-11β-phénylgonanes de formule générale I :

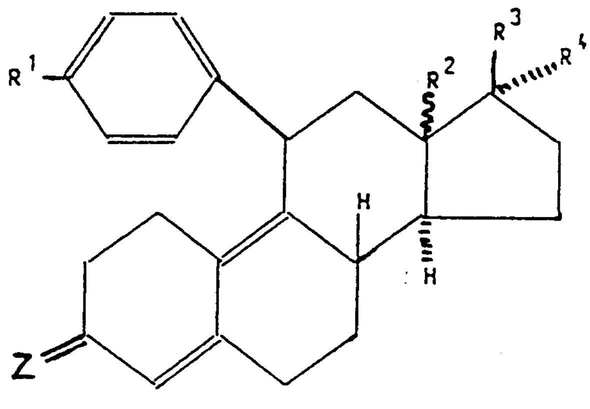

(I),

où

Z représente un atome d'oxygène ou un groupement hydroxyimino N~OH,

$R^1$ représente

a) soit un reste hétéroaryle de formule Ia :

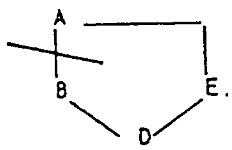

(Ia),

où A = N, O ou S et

B-D-E la série d'éléments C-C-C, N-C-C ou C-N-C, à l'exception du reste 1-pyrazole, soit

b) un reste hétéroaryle de formule Ib :

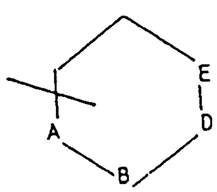

(Ib),

où A = N et

B-D-E la série d'éléments C-C-C, N-C-C, C-N-C ou C-C-N, soit

c) un reste Ic cycloalkyle, cycloalcényle ou aryle, soit

d) un reste alcényle Id linéaire ou ramifié, présentant une ou plusieurs doubles liaisons, avec 2 à 10 atomes de carbone,

et où éventuellement

le reste hétéroaryle de formule Ia peut être substitué par un ou plusieurs restes halogène et/ou un ou plusieurs restes alkyle avec 1 à 3 atomes de carbone, et le reste Ic cycloalkyle, cycloalcényle ou aryle par un ou plusieurs restes halogène, des restes alcoxy, hydroxy éventuellement protégés, restes dialkylamino et/ou alkylthio éventuellement oxydés sous forme sulfoxyde ou sulfone et/ou N-oxyde, et

$R^2$ représente un reste méthyle ou éthyle en position α ou β, lorsque $R^2$ est en position α,

$R^1$ représente de plus un reste alkyle linéaire ou ramifié avec 1 à 10 atomes de carbone et

$R^3/R^4$

$-OR_5/-C\equiv CY$ $\quad$ $-H/-\underset{\overset{\|}{O}}{C}-CH_2R_6$

$-C\equiv CY/-OR_5$ $\quad$ $-\underset{\overset{\|}{O}}{C}-CH_2R_6/H$

$-OR_5/-\underset{\overset{\|}{O}}{C}-CH_2R_6$ $\quad$ $-OR_5/-(CH_2)_o-CH_2R_7$

$-\underset{\overset{\|}{O}}{C}-CH_2R_6/-OR_5$ $\quad$ $-(CH_2)_o-CH_2R_7/-OR_5$

$-CH_3/-\underset{\overset{\|}{O}}{C}-CH_2R_6$ $\quad$ $-OR_5/-CH=CH-(CH_2)_kCH_2R_7$

$-\underset{\overset{\|}{O}}{C}-CH_2R_6/-CH_3$ $\quad$ $-CH=CH-(CH_2)_kCH_2R_7/-OR_5$

$-OR_5/-H$

$-H/-OR_5$

et

17 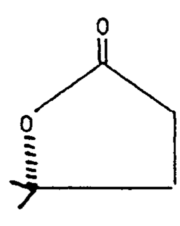 ou 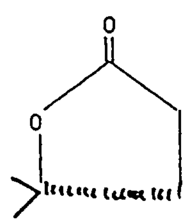

lorsque $R^2$ est en position $\beta$,
$R^1$ peut être de plus un radical éthyle, mais $R^1$ ne peut pas être le reste alcényle, isopropényle, et $R^3/R^4$

$-OR_5/-C\equiv CY$ $\qquad$ $\underset{\underset{O}{\|}}{C}-CH_2R_6/-H$

$-OR_5/-\underset{\underset{O}{\|}}{C}-CH_2R_6$ $\qquad$ $-OR_5/-(CH_2)_o-CH_2R_7$

$-\underset{\underset{O}{\|}}{C}-CH_2R_6/-OR_5$ $\qquad$ $-OR_5/-CH=CH-(CH_2)_kCH_2R_7$

$-OR_5/-H$

$-\underset{\underset{O}{\|}}{C}-CH_2R^6/-CH_3$

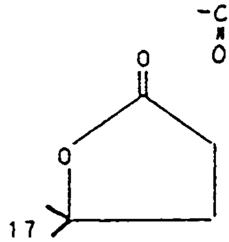

avec $R_5$ ayant la signification d'un atome d'hydrogène ou d'un reste acyle avec 1 à 4 atomes de carbone,
Y ayant la signification d'un atome d'hydrogène, de chlore, de fluor, d'iode ou de brome, d'un groupe alkyle, hydroxyalkyle, alcoxyalkyle ou acyloxyalkyle avec chaque fois 1 à 4 atomes de carbone dans le radical alkyle respectivement acyle,
$R_6$ dans la signification d'un atome d'hydrogène, d'un groupe hydroxy, d'un groupe alkyle, O-alkyle ou O-acyle avec chaque fois 1 à 4 atomes de carbone,
o valant 0, 1, 2 ou 3,
$R_7$ ayant la signification d'un reste hydroxy ou cyanure, d'un groupe O-alkyle ou O-acyle, avec chaque fois 1 à 4 atomes de carbone, et
k valant 0, 1 ou 2.

**2.** 11β-(4-Éthylphényl)-17-hydroxy-17β-(3-hydroxypropyl)-13α-méthyl-4,9-gonadién-3-one
11β-(4-éthylphényl)-17-hydroxy-17α-(3-hydroxypropyl)-13α-méthyl-4,9-gonadién-3-one
17-hydroxy-17β-(3-hydroxypropyl)-13α-méthyl-11β-(4-vinylphényl)-4,9-gonadién-3-one
17-hydroxy-17α-(3-hydroxypropyl)-13α-méthyl-11β-(4-vinylphényl)-4,9-gonadién-3-one
17-hydroxy-17β-(3-hydroxypropyl)-11β-(4-isopropénylphényl)-13α-méthyl-4,9-gonadién-3-one
17-hydroxy-17α-(3-hydroxypropyl)-11β-(4-isopropénylphényl)-13α-méthyl-4,9-gonadién-3-one
17-hydroxy-17β-(3-hydroxypropyl)-13a-méthyl-11β-[4-(3-thiényl)-phényl]-4,9-gonadién-3-one
17-hydroxy-17α-(3-hydroxypropyl)-13α-méthyl-11β-[4-(3-thiényl)-phényl]-4,9-gonadién-3-one
17-hydroxy-17β-(3-hydroxypropyl)-13α-méthyl-11β-[4-(2-thiényl)-phényl]-4,9-gonadién-3-one
17-hydroxy-17α-(3-hydroxypropyl)-13α-méthyl-11β-[4-(2-thiényl)-phényl]-4,9-gonadién-3-one
11β-[4-(2-furyl)-phényl]-17-hydroxy-17β-(3-hydroxypropyl)-13α-méthyl-4,9-gonadién-3-one
11β-[4-(3-furyl)-phényl]-17-hydroxy-17α-(3-hydroxypropyl)-13α-méthyl-4,9-gonadién-3-one
11β-[4-(3-furyl)-phényl]-17-hydroxy-17β-(3-hydroxypropyl)-13α-méthyl-4,9-gonadién-3-one
11β-[4-(3-furyl)-phényl]-17-hydroxy-17α-(3-hydroxypropyl)-13α-méthyl-4,9-gonadién-3-one
17-hydroxy-17β-(3-hydroxypropyl)-13α-méthyl-11β-[4-(2-pyridyl)-phényl]-4,9-gonadién-3-one
17-hydroxy-17α-(3-hydroxypropyl)-13α-méthyl-11β-[4-(2-pyridyl)-phényl]-4,9-gonadién-3-one
17-hydroxy-17β-(3-hydroxypropyl)-13α-méthyl-11β-[4-(3-pyridyl)-phényl]-4,9-gonadién-3-one
17-hydroxy-17α-(3-hydroxypropyl)-13α-méthyl-11β-[4-(3-pyridyl)-phényl]-4,9-gonadién-3-one
17-hydroxy-17β-(3-hydroxypropyl)-13α-méthyl-11β-[4-(4-pyridyl)-phényl]-4,9-gonadién-3-one
17-hydroxy-17α-(3-hydroxypropyl)-13α-méthyl-11β-[4-(4-pyridyl)-phényl]-4,9-gonadién-3-one
11β-[4-(4-diméthylaminophényl)-phényl]-17-hydroxy-17β-(3-hydroxypropyl-13α-méthyl-4,9-gonadién-3-one

11$\beta$-[4-(4-diméthylaminophényl)-phényl]-17-hydroxy-17$\alpha$-(3-hydroxypropyl-13$\alpha$-méthyl-4,9-gonadién-3-one
11$\beta$-[4-(3-diméthylaminophényl)-phényl]-17-hydroxy-17$\beta$-(3-hydroxypropyl-13$\alpha$-méthyl-4,9-gonadién-3-one
17-hydroxy-17$\beta$-(3-hydroxypropyl)-13$\alpha$-méthyl-11$\beta$-[4-(4-cyanophényl)-phényl]-4,9-gonadién-3-one
11$\beta$-[4-(3-diméthylaminophényl)-phényl]-17-hydroxy-17$\alpha$-(3-hydroxypropyl)-13$\alpha$-méthyl-4,9-gonadién-3-one
11$\beta$-[4-(2-diméthylaminophényl)-phényl]-17-hydroxy-17$\beta$-(3-hydroxypropyl)-13$\alpha$-méthyl-4,9-gonadién-3-one
11$\beta$-[4-(2-diméthylaminophényl)-phényl]-17-hydroxy-17$\alpha$-(3-hydroxypropyl)-13$\alpha$-méthyl-4,9-gonadién-3-one
17-hydroxy-17$\beta$-(3-hydroxypropyl)-13$\alpha$-méthyl-11$\beta$-[4-(2-thiazolyl)-phényl]-4,9-gonadién-3-one
17-hydroxy-17$\alpha$-(3-hydroxypropyl)-13$\alpha$-méthyl-11$\beta$-[4-(2-thiazolyl)-phényl]-4,9-gonadién-3-one
11$\beta$-(4-éthylphényl)-17-hydroxy-17$\alpha$-(3-hydroxyprop-(Z)-1-ényl)-4,9-estradién-3-one
17-hydroxy-17$\alpha$-(3-hydroxyprop-(Z)-1-ényl)-11$\beta$-(4-vinylphényl)-4,9-estradién-3-one
17-hydroxy-17$\alpha$-(3-hydroxyprop-(Z)-1-ényl)-11$\beta$-[4-(3-thiényl)-phényl]-4,9-estradién-3-one
17-hydroxy-17$\alpha$-(3-hydroxyprop-(Z)-1-ényl)-11$\beta$-[4-(2-thiényl)-phényl]-4,9-estradién-3-one
11$\beta$-(4-éthylphenyl)-17-hydroxy-17$\alpha$-(1-propynyl)-4,9-estradién-3-one
17-hydroxy-17$\alpha$-(1-propynyl)-11$\beta$-(4-vinylphényl)-4,9-estradién-3-one
17-hydroxy-17$\alpha$-(3-hydroxyprop-(Z)-1-ényl)-11$\beta$-[4-(3-furyl)-phényl]-4,9-estradién-3-one

**3.** 13-Alkylgonanes selon la revendication 1, dans lesquels le reste hétéroaryle de formule Ia représente les restes 3-thiényle, 3-furyle ou 3-pyrrolyle.

**4.** 13-Alkylgonanes selon la revendication 1, dans lesquels le reste hétéroaryle de formule Ib est le reste 3- ou 4-pyridyle, 5-pyrimidine, 4-pyridazine ou pyrazine.

**5.** 13-Alkylgonanes selon la revendication 1, dans lesquels le reste Ic cycloalkyle, cycloalcényle ou aryle est le reste cyclohexyle, un 1-cyclohexényle ou le reste phényle.

**6.** 13-Alkylgonanes selon la revendication 1, dans lesquels le reste alcényle Id présente jusqu'à trois doubles liaisons.

**7.** 13-Alkylgonanes selon la revendication 1, dans lesquels le reste hétéroaryle de formule Ia est substitué par un atome de chlore ou de brome.

**8.** 13-Alkylgonanes selon la revendication 1, dans lesquels le reste hétéroaryle de formule Ia est substitué par un reste alkyle avec 1 à 3 atomes de carbone.

**9.** 13-Alkylgonanes selon la revendication 1, dans lesquels le reste Ic cycloalkyle, cycloalcényle ou aryle est substitué par 1 ou 2 atomes de chlore et/ou de brome.

**10.** 13-Alkylgonanes selon la revendication 1, dans lesquels le reste Ic cycloalkyle, cycloalcényle ou aryle est substitué par 1 ou 2 restes hydroxy et/ou alcoxy avec 1 à 8 atomes de carbone éventuellement protégés.

**11.** Procédé pour la préparation de composés de formule générale I :

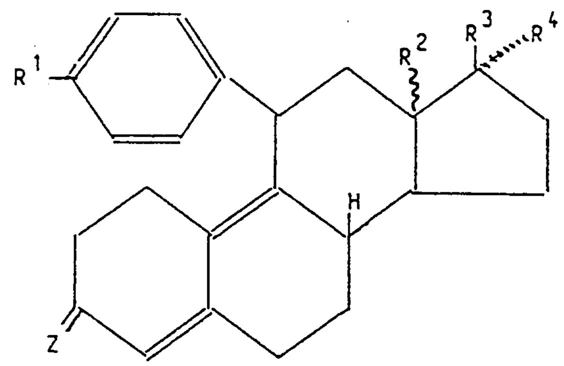

(I),

où

Z représente un atome d'hydrogène ou le groupement hydroxyimino N~OH,

$R^1$ représente

a) soit un radical hétéroaryle de formule Ia :

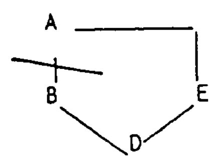

(Ia),

où A = N, O ou S et

B-D-E la série d'éléments C-C-C, N-C-C ou C-N-C, à l'exception du reste 1-pyrazole, soit

b) un reste hétéroaryle de formule Ib :

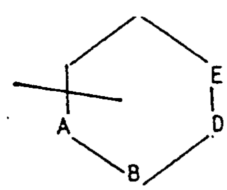

(Ib),

où A = N et

B-D-E la série d'éléments C-C-C, N-C-C, C-N-C ou C-C-N soit

c) un reste Ic cycloalkyle, cycloalcényle ou aryle, soit

d) un reste alcényle Id linéaire ou ramifié, présentant une ou plusieurs doubles liaisons, avec 2 à 10 atomes de carbone,

et où éventuellement

le reste hétéroaryle de formule Ia peut être substitué par un ou plusieurs restes halogène et/ou un ou plusieurs restes alkyle avec 1 à 3 atomes de carbone

et le reste Ic cycloalkyle, cycloalcényle ou aryle par un ou plusieurs restes halogène, restes alcoxy, hydroxy éventuellement protégés, restes dialkylamino et/ou alkylthio éventuellement oxydés sous forme sulfoxyde ou sulfone et/ou N-oxyde, et

$R^2$ représente un reste méthyle ou éthyle en position $\alpha$ ou $\beta$, lorsque $R^2$ est en position $\alpha$,

$R^1$ représente de plus un reste alkyle linéaire ou ramifié avec 1 à 10 atomes de carbone $R^3/R^4$

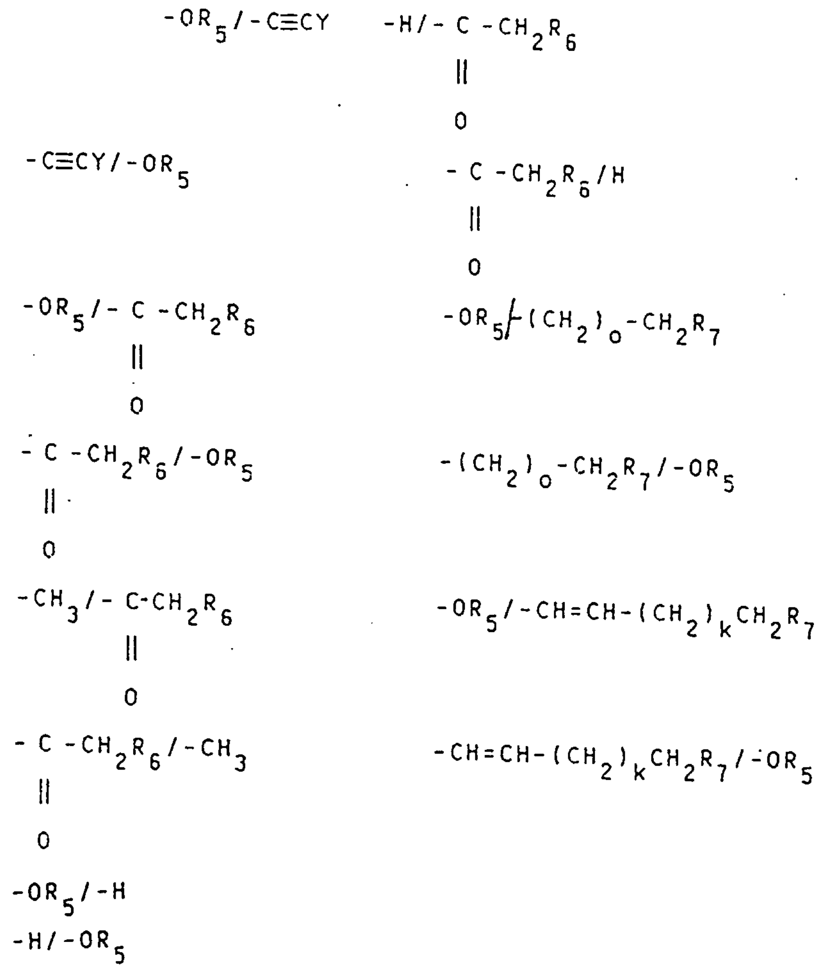

et

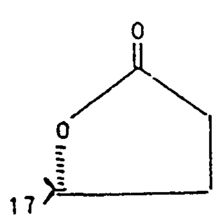

ou

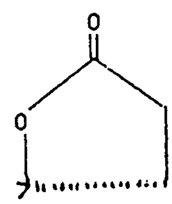

lorsque $R^2$ est en position $\beta$

$R^1$ peut être de plus un radical éthyle, mais $R^1$ ne peut pas être le reste alcényle, isopropényle, et $R^3/R^4$

$-OR_5/-C\equiv CY$

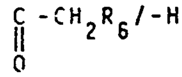

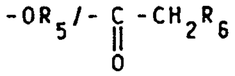

$-OR_5/-(CH_2)_o-CH_2R_7$

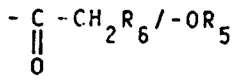

$-OR_5/-CH=CH-(CH_2)_kCH_2R_7$

$-OR_5/-H$

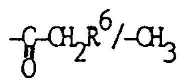

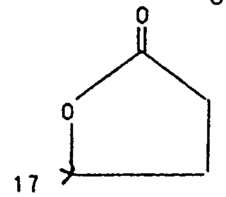

avec $R_5$ ayant la signification d'un atome d'hydrogène ou d'un reste acyle avec 1 à 4 atomes de carbone,
Y ayant la signification d'un atome d'hydrogène, de chlore, de fluor, d'iode ou de brome, d'un groupe alkyle, hydroxyalkyle, alcoxyalkyle ou acyloxyalkyle avec chaque fois 1 à 4 atomes de carbone dans le radical alkyle respectivement acyle,
$R_6$ dans la signification d'un atome d'hydrogène, d'un groupe hydroxy, d'un groupe alkyle, O-alkyle ou O-acyle avec chaque fois 1 à 4 atomes de carbone, o valant 0, 1, 2 ou 3,
$R_7$ ayant la signification d'un reste hydroxy ou cyanure, d'un groupe O-alkyle ou O-acyle avec chaque fois 1 à 4 atomes de carbone et
k valant 0, 1 ou 2,
caractérisé en ce qu'un composé de formule générale II :

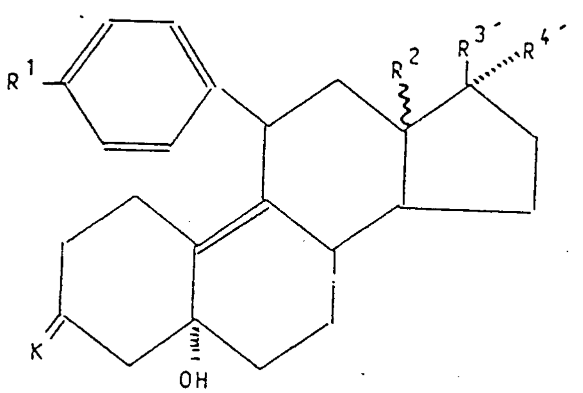

(II),

dans laquelle K représente un groupe céto bloqué sous forme du cétal, du thiocétal, de l'oxime ou du méthyloxime, $R^1$ et $R^2$ ont les significations données ci-dessus et $R^{3'}$ et $R^{4'}$ ont la même signification que $R^3$ ou bien $R^4$, les groupes hydroxyles éventuellement présents dans $R^3$ et les groupes hydroxyles et/ou acyles éventuellement présents dans $R^4$ étant protégés, est soumis à l'effet d'un agent déshydratant, qui est également apte à la libération des fonctions protégées, à des fins d'élimination d'eau en formant une double liaison 4(5), en protégeant à nouveau, si on le souhaite, les restes hydroxyle et/ou alcoxy éventuellement présents dans le reste Ic cycloalkyle, cycloalcényle ou aryle, en oxydant en sulfoxyde, sulfone et/ou N-oxyde correspondants les restes alkylthio et/ou dialkylamino éventuellement présents dans le reste Ic et, si on le souhaite, en faisant réagir ensuite avec le chlorhydrate d'hydroxylamine en présence d'amines tertiaires à des températures comprises entre -20 et +40 °C.

**12.** Procédé pour la préparation des produits de départ de formule générale II :

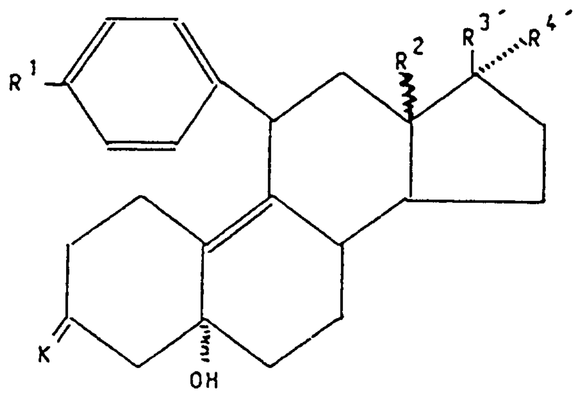

(II),

dans laquelle K, $R^1$, $R^2$, $R^{3'}$ et $R^{4'}$ ont les significations données, caractérisé en ce qu'on transforme un composé de formule générale III :

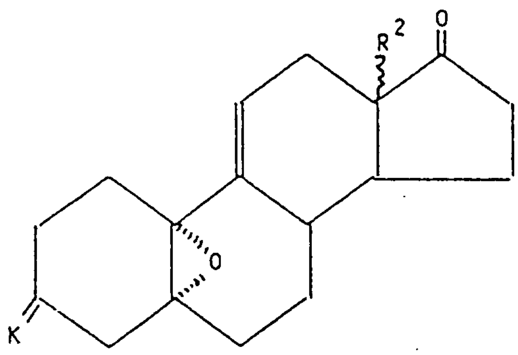

(III),

dans laquelle K et $R^2$ ont les significations données selon des procédés connus en soi de l'édification de la chaîne latérale C-17 en un composé de formule générale IV :

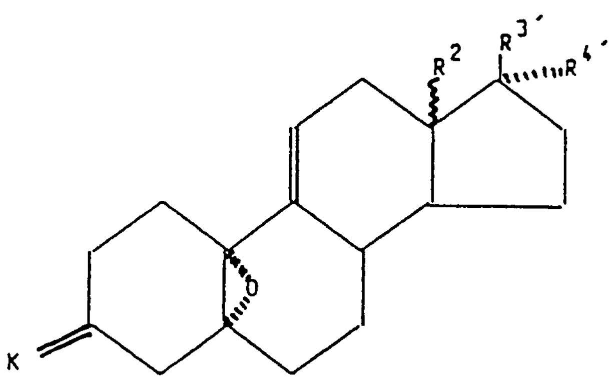

(IV),

dans laquelle K, $R^2$, $R^{3'}$ et $R^{4'}$ ont les significations déjà indiquées, et on transforme ensuite ce composé de formule générale IV est ensuite transformé soit par réaction de Grignard avec l'halogénoarylmagnésium correspondant

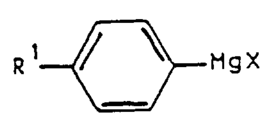

(X = Br, I) en un composé de formule générale II, soit par alkylation avec un composé du type

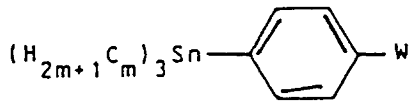

où W = MgX (X = Br, I) ou de préférence Li et m = 1, 2, 3, 4, de préférence 1 ou 4, en un composé de formule générale V :

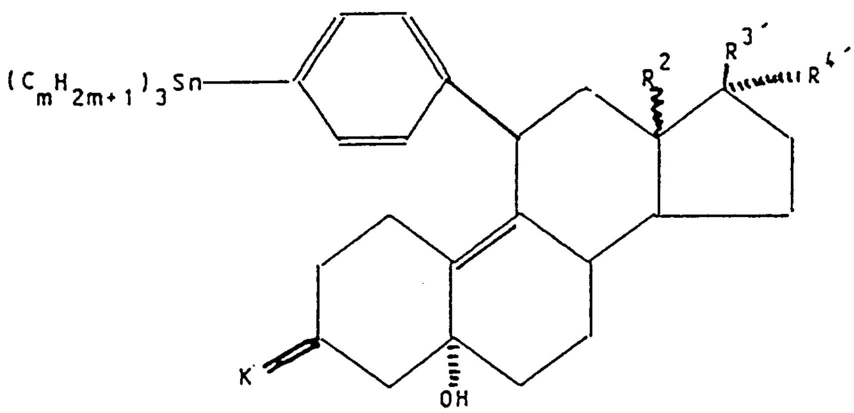

(V),

dans laquelle tous les substituants ont la signification déjà donnée,
on fait réagir ceux-ci ensuite par une réaction catalysée par un métal de transition, de préférence par Pd(O) en présence d'un composé $R^1$-Y où $R^1$ a la signification du substituant désiré dans le produit de départ de formule générale II et Y représente halogène, de préférence brome, en un composé de formule générale II ou
lorsque $R^2$ est en position $\alpha$ par réaction de Grignard avec l'halogénure d'halogénoarylmagnésium correspondant

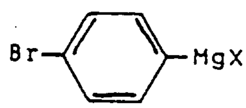

(X = Br, I) en un composé de formule générale VI :

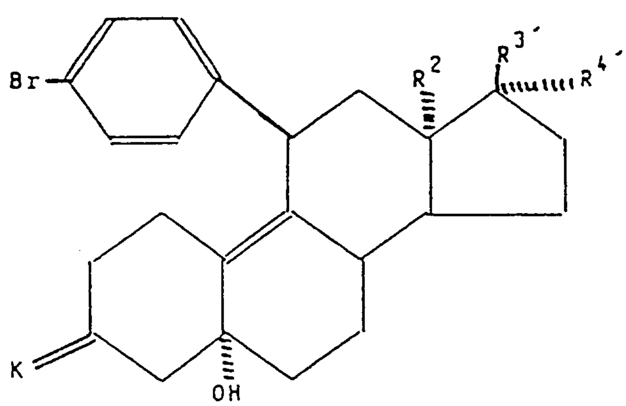

(VI),

dans laquelle K, $R^2$, $R^{3'}$ et $R^{4'}$ ont les significations données ci-dessus, et ensuite on fait réagir le composé de formule générale VI pour introduire le substituant $R^1$ dans la position 4 du cycle phényle

configuré en 11$\beta$ en catalysant à l'aide de métal de transition, de préférence de Pd(0), avec un composé organo-étain de formule générale $R^1Sn(C_mH_{2m+1})_3$ en formant un composé de formule générale II, ou bien on catalyse d'abord un compose de formule générale VI à l'aide de métaux de transition, de préférence à l'aide de Pd(0) avec un hexaalkyle-distannane $[(C_mH_{2m+1})_3Sn]_2$ (m = 1, 2, 3, 4, de préférence 1 ou 4), pour obtenir un composé de formule générale V puis on traite celui-ci ultérieurement - comme déjà indiqué - pour obtenir un composé de formule générale II.

**13.** Procédé pour la préparation de composés de formule générale II :

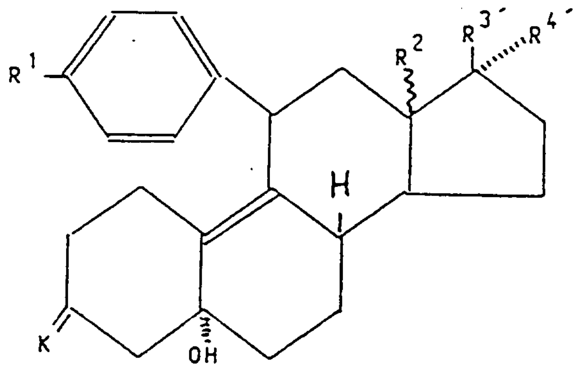

(II),

dans laquelle K, $R^1$, $R^2$, $R^{3'}$ et $R^{4'}$ ont les significations déjà données, caractérisé en ce qu'on alkyle d'abord un composé de formule générale VII :

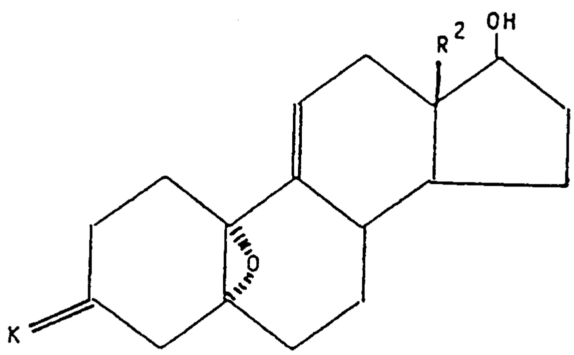

(VII),

dans laquelle K et $R^2$ ont la signification donnée précédemment avec un composé de formule

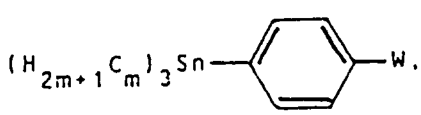

où W = MgX (X = Br, I) ou de préférence Li et
m = 1, 2, 3, 4, de préférence 1 ou 4, en position 11, en formant un composé de formule générale VIII :

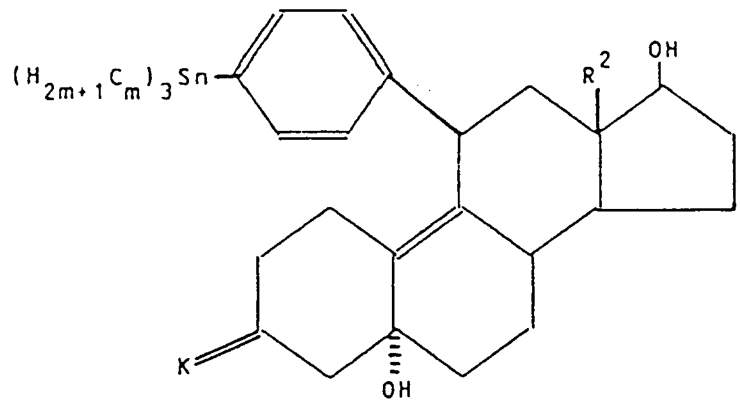

(VIII)

on fait réagir celui-ci par copulation catalysée par un métal de transition, de préférence par du Pd(0), avec un composé $R^1$-Y (Y = Br, I), pour obtenir un composé de formule générale IX :

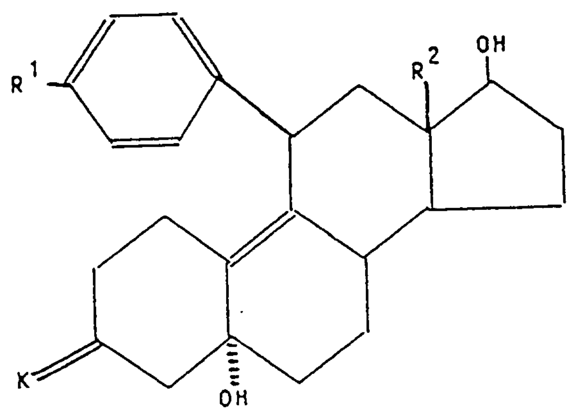

(IX),

et ensuite par oxydation de la fonction 17-$\beta$-OH et éventuellement par irradiation ultérieure avec de la lumière ultraviolette, on transforme en un composé de formule générale X :

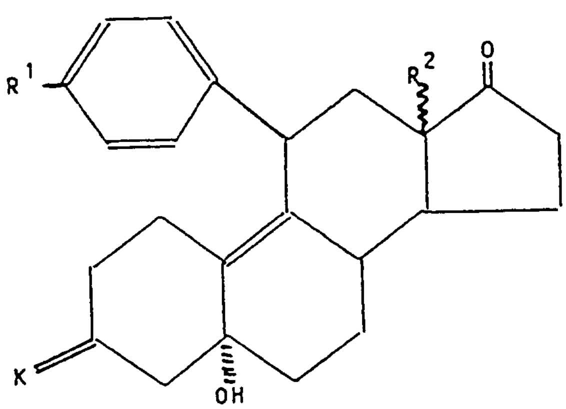

(X),

dans laquelle les substituants ont les significations déjà indiquées et $R^2$ pouvant être aussi bien en position $\alpha$ qu'en position $\beta$, et ensuite on incorpore dans X les substituants $R^3$ et $R^4$ ou encore $R^{3'}$ et/ou $R^{4'}$ par addition nucléophile (édification de la chaîne latérale) en formant un composé de formule générale II :

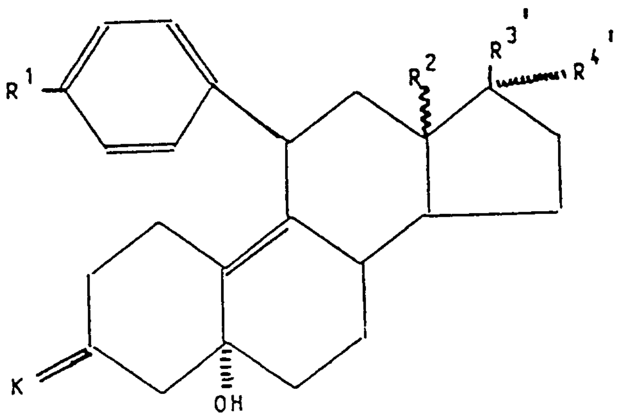

(II),

ou d'abord on oxyde un composé de formule générale VIII en position 17 pour obtenir un composé de formule générale XI :

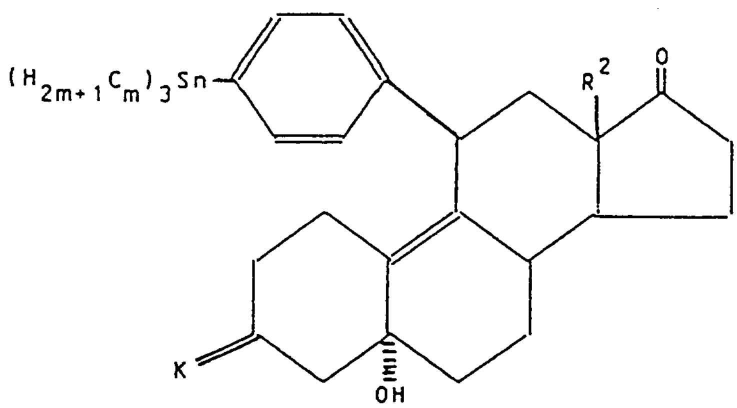

(XI),

et ensuite on traite celui-ci par addition nucléophile (édification de la chaîne latérale) éventuellement après irradiation par de la lumière ultraviolette, par l'intermédiaire d'un composé de formule générale V et par copulation catalysée par un métal de transition, de préférence par Pd(0), avec un composé $R^1$-Y (Y = Br, I) pour obtenir un composé de formule générale II.

**14.** Préparations pharmaceutiques caractérisées en ce qu'elles contiennent des composés selon les revendications 1 à 10.

**15.** Utilisation des composés selon les revendications 1 à 10 pour la préparation de médicaments.